# EUROPEAN PATENT SPECIFICATION

(11) **EP 2 992 094 B1**
(45) Date of publication and mention of the grant of the patent: **20.06.2018**
(21) Application number: 14791797.5
(22) Date of filing: 01.05.2014
(51) Int. Cl.: A61K 48/00, C07K 14/62, C12N 15/85

(54) **COMPOSITIONS AND METHODS FOR TREATMENT OF TYPE 1 DIABETES**
ZUSAMMENSETZUNGEN UND VERFAHREN ZUR BEHANDLUNG VON DIABETES VOM TYP 1
COMPOSITIONS ET PROCÉDÉS POUR LE TRAITEMENT DU DIABÈTE DE TYPE 1

(30) Priority: 02.05.2013 US 201361818671 P
(43) Date of publication of application: 09.03.2016
(73) Proprietor: Tolerion, Portola Valley, CA 94028 (US)
(72) Inventor: ROEP, Bart, O., Portola Valley, CA 94028 (US); ROBINSON, William, H., Menlo Park, CA (US); UTZ, Paul, Portola Valley, CA (US); GARREN, Hideki, Palo Alto, CA 94306 (US); STEINMAN, Lawrence, Stanford, CA 94304 (US)
(74) Representative: Harrison, Susan Joan
(86) International application number: PCT/US2014/036394
(87) International publication number: WO 2014/179586

(56) References cited:
- WO-A1-2010/151420
- WO-A1-2012/015903
- WO-A2-02/45735
- US-A1- 2012 035 105
- BART O ROEP ET AL: "Plasmid-Encoded Proinsulin Preserves C-Peptide While Specifically Reducing Proinsulin-Specific CD8+ T Cells in Type 1 Diabetes", SCIENCE TRANSLATIONAL MEDICINE, AMERICAN ASSOCIATION FOR THE ADVANCEMENT OF SCIENCE, WASHINGTON, DC, vol. 5, no. 191, 26 June 2013 (2013-06-26) , XP009170924, ISSN: 1946-6234, DOI: 10.1126/SCITRANSLMED.3006103

## Description

### FIELD OF THE INVENTION

This invention relates to compositions for use in methods for treating insulin-dependent diabetes mellitus in a subject.

### BACKGROUND OF THE INVENTION

One of the hallmarks of Type 1 diabetes (T1D) is an inflammatory response that ultimately destroys the beta cells of the pancreas, a process termed insulitis. CD8 T cells directed to various islet antigens including preproinsulin (PPI), glutamic acid decarboxylase (GAD), tyrosine phosphatase-like insulinoma antigen (IA2, also called ICA512), zinc transporter ZnT8, and islet specific glucose-6-phosphatase catalytic subunit related protein (IGRP) have been detected in the blood and in the pancreatic islets of individuals with T1D (*1-3*). Thus, attempts have been made to use antigen-specific therapy to delay T1D, including parenterally and nasally administered insulin (*4-6*). However, a trial of oral insulin failed to delay T1D, although there was evidence of delay in a subset of patients with high levels of insulin autoantibodies (*6,7*). Other clinical trials targeting GAD with alum were unsuccessful in Phase 3 in reducing loss of C-peptide-a marker of beta cell function, possibly due to the use of an adjuvant that failed to show efficacy in murine models of T1D (*8*). In contrast, a recent Phase 3 trial of a heat shock peptide (DiaPep277) reported successful outcomes for preservation of C-peptide, insulin usage, and HgbAlc (9). Importantly, these trials involving injection of self-molecules have demonstrated safety, with no serious adverse events reported to date.

One approach that was successful in preclinical experiments in mouse models of T1D, was using an engineered DNA vaccine encoding the whole proinsulin molecule, including C-peptide, insulin A and B chains (*10-12*). Tolerization to proinsulin prevented and reversed active insulitis in hyperglycemic nonobese diabetic (NOD) mice, a widely studied mouse model of T1D (*12*).

Adaptive immune responses to islet-associated antigens have been identified in T1D. Pancreatic specimens obtained from T1D patients reveal a lymphocytic infiltrate in the pancreatic islets, composed predominantly of CD8+ T cells, with upregulated HLA class I molecules (*1,14*). These findings suggest a key pathophysiologic role for cytotoxic T lymphocytes in T1D. CD4+ T cells are also likely involved in the pathogenesis of T1D, further supported by the strong association of susceptibility in T1D with certain HLA class II haplotypes (*14*). Finally, autoantibodies to pancreatic islet antigens have been found in the overwhelming majority of T1D patients and those at genetic risk for developing the disease. Antibodies to either GAD, IA2 or insulin, are present in 95% of pre-diabetic or new-onset T1D patients; 80% of patients are positive for two or more of these antibodies, and 25% are positive have all three antibodies. Multiple T1D-associated autoantibodies are present rarely in serum of healthy control subjects (3).

Insulin is a primary β cell-specific autoantigen, and insulin autoantibodies are usually the first to appear in young children with T1D (*3,15*). Furthermore, half of the T cells isolated from pancreatic draining lymph nodes of patients with T1D recognize an epitope of the insulin A chain, whereas T cells from healthy subjects that recognize this epitope have not been observed (*16*). Finally, it has been demonstrated that insulin-reactive T cells from T1D patients exhibit an activated inflammatory Th1 cell phenotype, whereas insulin-reactive T cells from healthy controls exhibit a protective T regulatory phenotype (*17*).

Methods for treating autoimmune disease by administering a nucleic acid encoding one or more autoantigens have been described, for example, in International Patent Application Nos. WO 00/53019, WO 2003/045316, and WO 2004/047734. While these methods have been successful, further improvements are still needed.

### BRIEF SUMMARY OF THE INVENTION

The present invention provides compositions for use in methods for treating or preventing insulin-dependent diabetes mellitus in a subject the composition being.

a self-vector having a nucleic acid sequence at least 95% identical to SEQ ID NO:1 (BHT-3021) for use in reducing the frequency of CD8 T cells reactive to proinsulin.

In some embodiments, the self-vector is administered in a pharmaceutically acceptable carrier or excipient.

Other objects, features, and advantages of the present invention will be apparent to one of skill in the art from the following detailed description and figures.

### BRIEF DESCRIPTION OF THE DRAWINGS

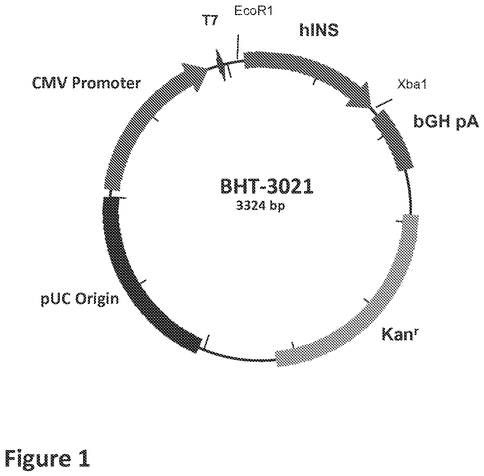

Figure 1 is a Structural Diagram of BHT-3021. BHT-3021 is a 3.3 Kb bacterial plasmid expression vector containing the coding sequences for human proinsulin (hINS) gene. Important functional and control features of BHT-3021 include the human cytomegalovirus (CMV) immediate-early gene promoter/enhancer, the bovine growth hormone gene polyadenylation signal, the kanamycin resistance gene, and pUC origin of replication for propagation of the vector in *E. coli.* The backbone of BHT-3021 has been modified to decrease the number of immunostimulatory CpG sequences and substitute immunosuppressive sequences.

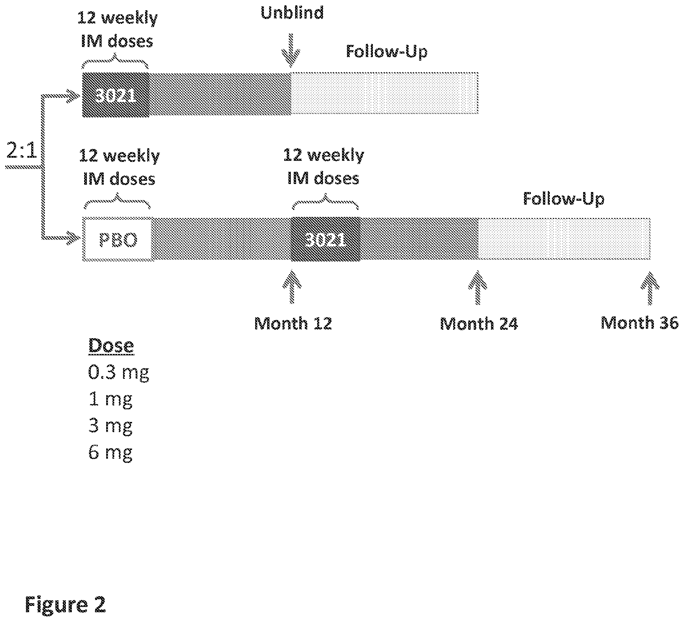

Figure 2 shows A Schematic of the Study Trial Design. Eighty subjects were enrolled in the study. Four dose levels of BHT-3021 were evaluated: 0.3 mg, 1.0 mg, 3.0 mg, and 6.0 mg. After completion of the dose-finding phase of the study (Dose Escalation Phase), additional subjects were enrolled to expand select dose cohorts in order to obtain additional safety and efficacy data (Expansion Phase). The dose escalation portion of the study enrolled subjects sequentially into the 1 mg, and then the 3 mg cohorts (randomized active:placebo 2:1); the cohorts for the 0.3 mg and 6 mg dose levels were then enrolled concurrently. After the dose escalation enrollment was complete, subjects were randomized (active:placebo 2:1) into the Expansion Cohort to receive BHT-3021 at doses of 0.3 mg, 1 mg, or 3 mg, or BHT-placebo.

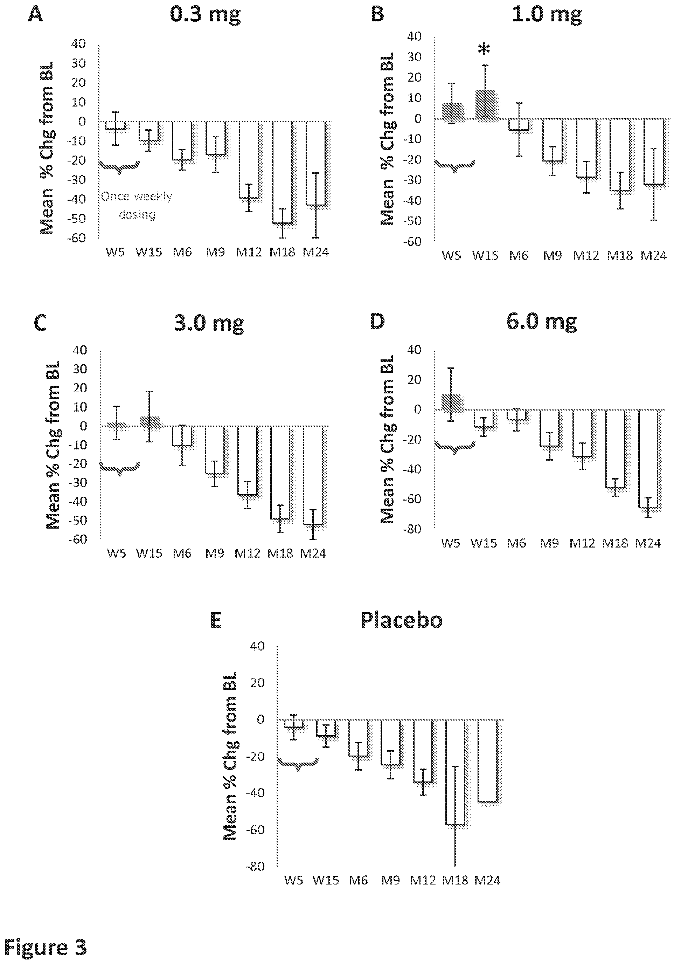

Figure 3 shows Mean Percent Change in C-Peptide from Baseline. C-peptide was assessed to determine whether there was not a precipitous fall in this measure of beta cell function during the 12 weekly doses and thereafter. C-peptide measured as described in methods (*18-20*). N=14 for 0.3 mg dose; n=15 for 1.0 mg dose; n=13 for 3.0mg dose; n=8 for 6.0 mg dose; n=23 for placebo. The mean percentage change from baseline +/- CI is displayed. W refers to week following initiation of 12-weekly doses at time zero, whereas M refers to month following initiation of 12-weekly doses at time zero.

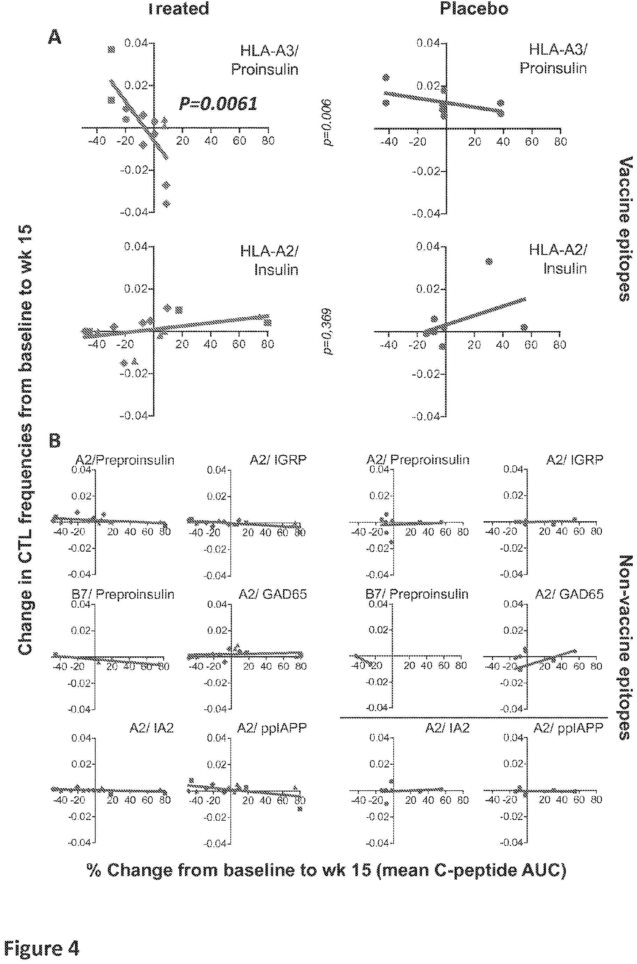

Figure 4 shows Antigen specific CD8 T cells were enumerated with Qdot-multimer technology using class I HLA multimers loaded with various antigens 21-24. Antigen and HLA haplotype are shown in each panel. CTL frequencies are defined as percentage of antigen-specific CD8 T cells. Changes in CTL from baseline to week 15 are shown on the y-axis, and % change in C-peptide from baseline at week 15 is shown on the x-axis. Changes in CTL were calculated by subtracting the baseline values from values at week 15. Analysis was performed on all treated (0.3 mg: diamonds, 1 mg: triangles, 3 mg: squares, 6 mg: circles) and placebo patients positive for HLA-A2, -A3 and/or -B7 (A) and for control antigens (B). Statistics were performed using linear regression analysis.

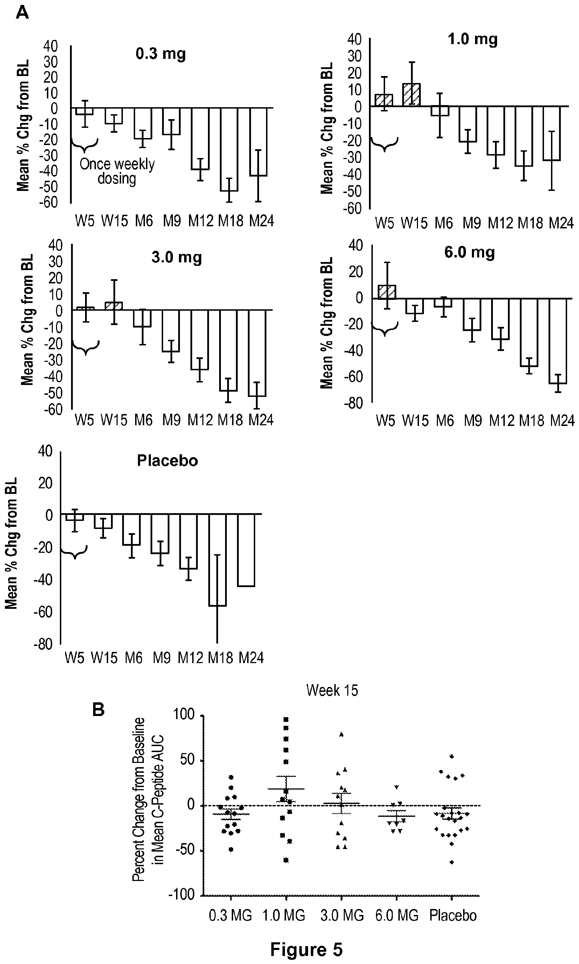

Figure 5 (A) shows Mean Percent Change in C-Peptide from Baseline over 24 Months. C-peptide was measured as described in methods (*18-20*). Through 12 months N=14 for 0.3 mg dose; n=15 for 1.0 mg dose; n=13 for 3.0mg dose; n=8 for 6.0 mg dose; n=23 for placebo. For 0.3 mg N=14 at M18 and N=7 at M24; 1.0 mg N=13 at M18 and N=7 at Month 24; 3mg N=12 at M18, N=9 at M24; 6.0 mg N=7 at M18 and n=6 at M24; Placebo n=3 at M18 and N=1 at M24. The mean percentage change from baseline +/- CI is displayed. Figrue 5(B) is a Scatter plot to visualize the individual patient's mean change in C-peptide levels at all doses at 15 weeks.

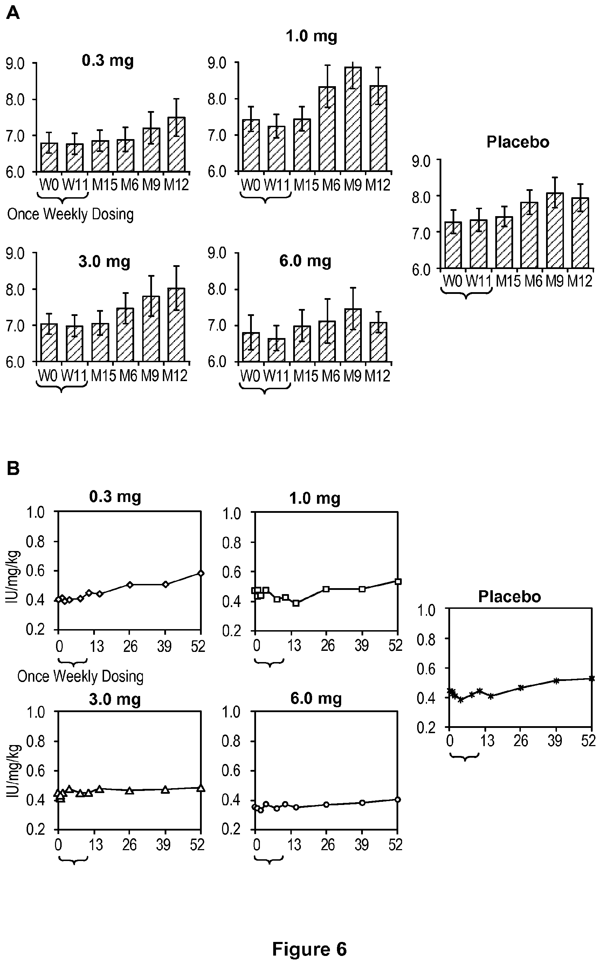

Figure 6 shows HgbAlc and Mean Total Insulin Usage at Various Doses of BHT-3021 vs BHT-Placebo. (A) Hemoglobin A1C is shown on the y axis, and time in months (M) on the x axis. (B) Mean total insulin usage in IU/mg/kg is depicted on the y axis, and time in weeks on the x axis. The 104 week data are not statistically significant, and this is now mentioned in the text. Further N=3 at week 104, as mentioned in the figure legend for Figure 5A.

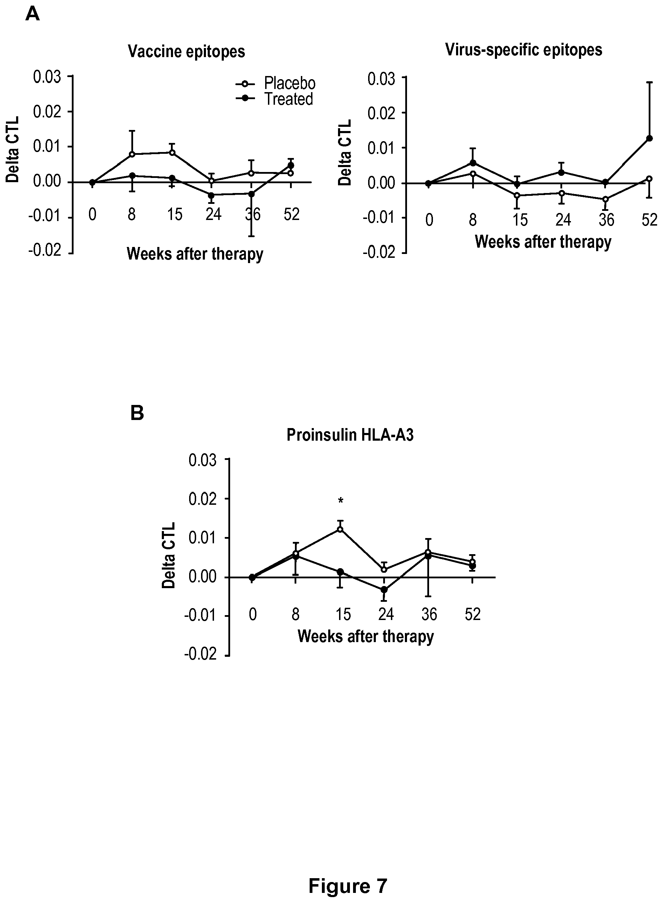

Figure 7 shows Changes in Antigen Specific T Cells over Time for Vaccine Related and Viral Related Epitopes. Antigen specific CD8 T cells against vaccine related epitopes (Proinsulin and Insulin) and virus-specific epitopes (EBV, CMV and measles); or (B) Proinsulin alone were enumerated with peptide-HLA multimers at baseline and weeks 8, 15, 24, 36 and 52. Deltas were calculated by subtracting the baseline values from values at each time point.

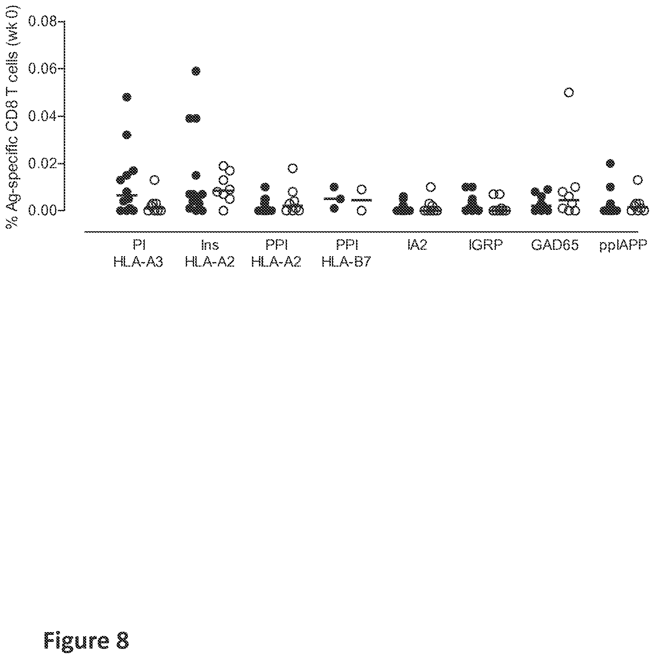

Figure 8 shows Frequencies of antigen-specific CD8 T-cells in treated (closed circles) and placebo (open circles) patients at week 0. Antigen-specific CD8 T cells were enumerated with Qdot-multimer technology using class I HLA multimers loaded with various antigens21-24. CTL frequencies are defined as percentage of antigen-specific CD8 T cells. PI: Proinsulin; Ins: Insulin; PPI: Preproinsulin.

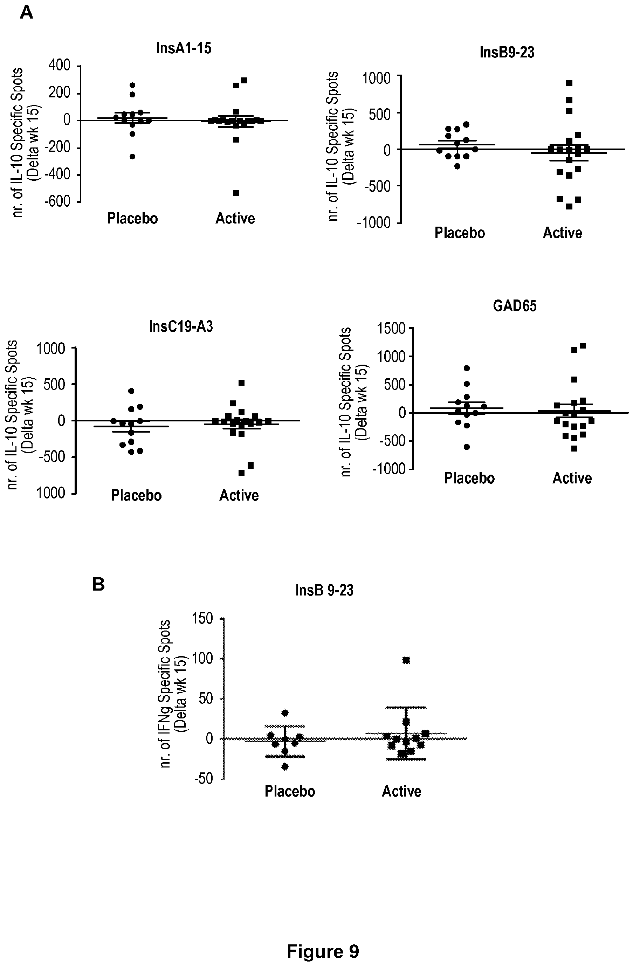

Figure 9 shows Number of IL-10 and Interferon-gamma Positive Elispots Compared to Baseline for 4 Different Antigen-Specific T Cell Subgroups. Change in the number of IL-10 (A) and interferon gamma (B) specific spots is measured on the y axis, comparing changes between week 0 and week 15, both for BHT-placebo and for BHT-3021 treated subjects.

### DETAILED DESCRIPTION

In Type 1 diabetes there is an intense inflammatory response that destroys the beta cells in the islets of langerhans in the pancreas, the site were insulin is produced and released. Proinsulin is a major target of the adaptive immune response in Type 1 Diabetes (T1D). The present invention provides an engineered DNA plasmid encoding proinsulin (BHT-3021) that preserves beta cell function in T1D patients through reduction of insulin-specific CD8 T cells. BHT-3021 is designed to decrease the antigen-specific autoimmune response against proinsulin in T1D. The plasmid was engineered with reduced numbers of pro-inflammatory hexa-nucleotide motifs, termed CpG motifs. CpG hexanucleotide sequences activate innate immune responses by binding to Toll Like Receptor 9 (TLR9) and other DNA sensors (13). All nonessential CpG sequences were replaced with GpG motifs, which compete with CpG motifs. This antigen-specific plasmid vaccine approach has the theoretical advantage of decreasing the autoimmune response while leaving intact other important, desirable, physiologic roles of the immune system, such as immune regulatory responses against proinsulin, immune surveillance against tumors, and immune responses against infectious agents.

The present invention provides methods of treating, reducing, preventing, and inhibiting insulin-dependent diabetes mellitus (IDDM) by administration of a self-vector encoding and capable of expressing human proinsulin. As described above, in IDDM, prior to the onset of overt diabetes, there is a long presymptomatic period during which there is a gradual loss of pancreatic β cell function. Markers that can be evaluated include without limitation blood or serum levels of C-peptide as indicative of pancreatic β cell function, the presence of insulitis in the pancreas, the level and frequency of islet cell antibodies, islet cell surface antibodies, the presence and concentration of autoantibodies against autoantigens targeted in IDDM, aberrant expression of Class II MHC molecules on pancreatic beta cells, glucose concentration in the blood, and the plasma concentration of insulin. An increase in the number of T lymphocytes in the pancreas, islet cell antibodies and blood glucose is indicative of the disease, as is a decrease in insulin concentration.

The Non-Obese Diabetic (NOD) mouse is an animal model with many clinical, immunological, and histopathological features in common with human IDDM. NOD mice spontaneously develop inflammation of the islets and destruction of the β cells, which leads to hyperglycemia and overt diabetes. Both CD4⁺ and CD8⁺ T cells are required for diabetes to develop, although the roles of each remain unclear. It has been shown that administration of insulin or GAD, as proteins, under tolerizing conditions to NOD mice prevents disease and down-regulates responses to the other autoantigens.

The presence of combinations of autoantibodies with various specificities in serum are highly sensitive and specific for human type I diabetes mellitus. For example, the presence of autoantibodies against GAD and/or IA-2 is approximately 98% sensitive and 99% specific for identifying type I diabetes mellitus from control serum. In non-diabetic first degree relatives of type I diabetes patients, the presence of autoantibodies specific for two of the three autoantigens including GAD, insulin and IA-2 conveys a positive predictive value of >90% for development of type IDM within 5 years.

Autoantigens targeted in human insulin dependent diabetes mellitus include, for example, insulin autoantigens, including insulin, insulin B chain, proinsulin, and preproinsulin; tyrosine phosphatase IA-2; IA-2β; glutamic acid decarboxylase (GAD) both the 65 kDa and 67 kDa forms; carboxypeptidase H; heat shock proteins (HSP); glima 38; islet cell antigen 69 KDa (ICA69); p52; two ganglioside antigens (GT3 and GM2-1); islet-specific glucose-6-phosphatase-related protein (IGRP); and an islet cell glucose transporter (GLUT 2).

Accordingly, the present invention provides methods for treating, preventing, reducing, inhibiting and/or delaying, *e.g.,* the symptoms of or the severity of IDDM in a subject comprising administration of the modified self-vector encoding and capable of expressing human proinsulin having a nucleic acid sequence at least 95% the identical to BHT-3021 (SEQ ID NO:1) Administration of a therapeutically or prophylactically effective amount of the modified self-vector to a subject elicits suppression of an immune response against an autoantigen associated with IDDM, thereby treating or preventing the disease. The self-vector may be co-administered or co-formulated with one or more divalent cations present at higher than physiologic concentrations. Surprisingly, co-administration of the self-vector with one or more divalent cations at total concentration higher than physiologic levels improves one or more of transfection efficiency, expression (*i.e.,* transcription and translation) of the encoded autoantigen, and therapeutic suppression of an undesirable immune response in comparison to co-administration of a self-vector in the presence of one or more divalent cations at total concentration equal to or lower than physiologic levels.

### II. Definitions

Unless defined otherwise, all technical and scientific terms used herein have the same meaning as commonly understood by those of ordinary skill in the art to which this invention belongs. The following references provide one of skill with a general definition of many of the terms used herein: Hale and Margham, The Harper Collins Dictionary of Biology (HarperPerennial, 1991); King and Stansfield, A Dictionary of Genetics (Oxford University Press, 4th ed. 1990); Stedman's Medical Dictionary (Lippincott Williams & Wilkins, 27th ed. 2000); and Hawley's Condensed Chemical Dictionary (John Wiley & Sons, 13th ed. 1997). As used herein, the following terms and phrases have the meanings ascribed to them unless specified otherwise.

The terms "intron" or "intronic sequence" as used herein refers to intervening polynucleotide sequences within a gene or portion of a gene present in a self-vector that is situated upstream of or between "exons", polynucleotide sequences that are retained during RNA processing and most often code for a polypeptide. Introns do not function in coding for protein synthesis and are spliced out of a RNA before it is translated into a polypeptide.

"Enhancer" refers to cis-acting polynucleotide regions of about from 10-300 basepairs that act on a promoter to enhance transcription from that promoter. Enhancers are relatively orientation and position independent and can be placed 5' or 3' to the transcription unit, within introns, or within the coding sequence itself.

The terms "DNA vaccination", "DNA immunization", and "polynucleotide therapy" are used interchangeably herein and refer to the administration of a polynucleotide to a subject for the purpose of modulating an immune response. "DNA vaccination" with plasmids expressing foreign microbial antigens is a well known method to induce protective antiviral or antibacterial immunit. For the purpose of the present invention, "DNA vaccination", "DNA immunization", or "polynucleotide therapy" refers to the administration of polynucleotides encoding one or more self-polypeptides that include one or more autoantigenic epitopes associated with a disease. The "DNA vaccination" serves the purpose of modulating an ongoing immune response to suppress autoimmune destruction for the treatment or prevention of an autoimmune disease. Modulation of an immune response in reaction to "DNA vaccination" may include shifting self-reactive lymphocytes from a Th1- to a Th2-type response. The modulation of the immune response may occur systemically or only locally at the target organ under autoimmune attack.

"Self- vector" means one or more vector(s) which taken together comprise a polynucleotide either DNA or RNA encoding one or more self-protein(s), -polypeptide(s), -peptide(s), *e.g.,* one or more autoantigens. Polynucleotide, as used herein is a series of either deoxyribonucleic acids including DNA or ribonucleic acids including RNA, and their derivatives, encoding a self-protein, -polypeptide, or -peptide of this invention. The self-protein, -polypeptide or -peptide coding sequence is inserted into an appropriate plasmid expression self-cassette. Once the polynucleotide encoding the self-protein, -polypeptide, or -peptide is inserted into the expression self-cassette the vector is then referred to as a "self-vector." In the case where polynucleotide encoding more than one self-protein(s), -polypeptide(s), or -peptide(s) is to be administered, a single self-vector may encode multiple separate self -protein(s), -polypeptide(s) or -peptide(s). In one embodiment, DNA encoding several self-protein(s), -polypeptide(s), or -peptide(s) are encoded sequentially in a single self-plasmid utilizing internal ribosomal re-entry sequences (IRES) or other methods to express multiple proteins from a single DNA molecule. The DNA expression self-vectors encoding the self-protein(s), -polypeptide(s), or -peptide(s) are prepared and isolated using commonly available techniques for isolation of plasmid DNA such as those commercially available from Qiagen Corporation. The DNA is purified free of bacterial endotoxin for delivery to humans as a therapeutic agent. Alternatively, each self-protein, -polypeptide or -peptide is encoded on a separate DNA expression vector.

The term "vector backbone" refers to the portion of a plasmid vector other than the sequence encoding a self-antigen, -protein, -polypeptide, or -peptide.

"Autoantigen," as used herein, refers to an endogenous molecule, typically a protein or fragment thereof, that elicits a pathogenic immune response. When referring to the autoantigen or epitope thereof as "associated with an autoimmune disease," it is understood to mean that the autoantigen or epitope is involved in the pathophysiology of the disease either by inducing the pathophysiology (*i.e.,* associated with the etiology of the disease), mediating or facilitating a pathophysiologic process; and/or by being the target of a pathophysiologic process. For example, in autoimmune disease, the immune system aberrantly targets autoantigens, causing damage and dysfunction of cells and tissues in which the autoantigen is expressed and/or present. Under normal physiological conditions, autoantigens are ignored by the host immune system through the elimination, inactivation, or lack of activation of immune cells that have the capacity to recognize the autoantigen through a process designated "immune tolerance."

As used herein the term "epitope" is understood to mean a portion of a polypeptide having a particular shape or structure that is recognized by either B-cells or T-cells of the animal's immune system. "Autoantigenic epitope" or "pathogenic epitope" refers to an epitope of an autoantigen that elicits a pathogenic immune response.

"Self-protein," "self-polypeptide," self-peptide," or "autoantigen" are used herein interchangeably and refer to any protein, polypeptide, or peptide, or fragment or derivative thereof that: is encoded within the genome of the animal; is produced or generated in the animal; may be modified posttranslationally at some time during the life of the animal; and, is present in the animal non-physiologically.

"Modulation of," "modulating", or "altering an immune response" as used herein refers to any alteration of an existing or potential immune responses against self-molecules, including, e.g., nucleic acids, lipids, phospholipids, carbohydrates, self-polypeptides, protein complexes, or ribonucleoprotein complexes, that occurs as a result of administration of a polynucleotide encoding a self-polypeptide. Such modulation includes any alteration in presence, capacity, or function of any immune cell involved in or capable of being involved in an immune response. Immune cells include B cells, T cells, NK cells, NK T cells, professional antigen-presenting cells, non-professional antigen-presenting cells, inflammatory cells, or any other cell capable of being involved in or influencing an immune response. "Modulation" includes any change imparted on an existing immune response, a developing immune response, a potential immune response, or the capacity to induce, regulate, influence, or respond to an immune response. Modulation includes any alteration in the expression and/or function of genes, proteins and/or other molecules in immune cells as part of an immune response.

"Modulation of an immune response" includes, for example, the following: elimination, deletion, or sequestration of immune cells; induction or generation of immune cells that can modulate the functional capacity of other cells such as autoreactive lymphocytes, antigen presenting cells (APCs), or inflamatory cells; induction of an unresponsive state in immune cells (*i.e.,* anergy); increasing, decreasing, or changing the activity or function of immune cells or the capacity to do so, including but not limited to altering the pattern of proteins expressed by these cells. Examples include altered production and/or secretion of certain classes of molecules such as cytokines, chemokines, growth factors, transcription factors, kinases, costimulatory molecules, or other cell surface receptors; or any combination of these modulatory events.

"Subjects" shall mean any animal, such as, for example, a human, non-human primate, horse, cow, dog, cat, mouse, rat, guinea pig or rabbit.

"Treating," "treatment," or "therapy" of a disease or disorder shall mean slowing, stopping or reversing the disease's progression, as evidenced by decreasing, cessation or elimination of either clinical or diagnostic symptoms, by administration of a polynucleotide encoding a self-polypeptide, either alone or in combination with another compound as described herein. "Treating," "treatment," or "therapy" also means a decrease in the severity of symptoms in an acute or chronic disease or disorder or a decrease in the relapse rate as for example in the case of a relapsing or remitting autoimmune disease course or a decrease in inflammation in the case of an inflammatory aspect of an autoimmune disease. In some embodiments, treating a disease means reversing or stopping or mitigating the disease's progression, ideally to the point of eliminating the disease itself. As used herein, ameliorating a disease and treating a disease are equivalent.

"Preventing," "prophylaxis," or "prevention" of a disease or disorder as used in the context of this invention refers to the administration of a polynucleotide encoding a self-polypeptide, either alone or in combination with another compound as described herein, to prevent the occurrence or onset of a disease or disorder or some or all of the symptoms of a disease or disorder or to lessen the likelihood of the onset of a disease or disorder.

A "therapeutically or prophylactically effective amount" of a self-vector refers to an amount of the self-vector that is sufficient to treat or prevent the disease as, for example, by ameliorating or eliminating symptoms and/or the cause of the disease. For example, therapeutically effective amounts fall within broad range(s) and are determined through clinical trials and for a particular patient is determined based upon factors known to the skilled clinician, including, *e.g*., the severity of the disease, weight of the patient, age, and other factors.

The phrase "endotoxin-free" refers to a vector or a composition of the invention that is substantially free of endotoxin, *e.g*., has endotoxin contamination below detectable levels. A vector or composition that is endotoxin-free can be described in terms of a threshold concentration of detectable endotoxin as measured using a Limulus Amebocyte Lysate (LAL) gel clot assay, known in the art. With respect to a threshold concentration, a vector or composition is endotoxin-free if the amount of contaminating endotoxin is below the limit of detection (*e.g*., less than about 0.10 endotoxin units/ml or EU/ml). To the extent that endotoxin can be detected, a vector or composition is substantially endotoxin-free if the amount of contaminating endotoxin is below about 2.5 EU/ml. Numerous companies provide commercially available testing services to determine the level of endotoxin in a preparation, including *e.g*., Nelson Laboratories, Salt Lake City, UT; Boehringer Ingelheim, Austria; MO BIO Laboratories, Carlsbad, CA; NovaTX, Conroe, TX; and Associates of Cape Cod, Inc., East Falmouth, MA. LAL gel clot detection kits are also available for purchase, from for example, Lonza, on the worldwide web at lonza.com and Charles River Laboratories, on the worldwide web at criver.com.

### III. Descriptions of the Embodiments

### A. BHT-3021 Self-Vector

In some embodiments, the present invention uses a self-vector having a nucleic acid sequence at least 95% identical to SEQ ID NO:1 (BHT-3021). The self-vector BHT-3021 comprises a BHT-1 expression vector backbone and a polynucleotide encoding human proinsulin. The self-vector BHT-3021 also comprises a CMV promoter, which drives the expression of human proinsulin; bovine growth hormone termination and polyA sequences; and a pUC origin of replication and a Kanamycin resistance gene (Kanr), which accomplish vector propagation and selection, respectively.

The backbone of the BHT-3021 vector is a modified pVAX1 vector in which one or more CpG dinucleotides of the formula 5'-purine-pyrimidine-C-G-pyrimidine-pyrimidine-3' is mutated by substituting the cytosine of the CpG dinucleotide with a non-cytosine nucleotide. The pVAX1 vector is known in the art and is commercially available from Invitrogen (Carlsbad, CA). In one exemplary embodiment, the modified pVAX1 vector has the following cytosine to non-cytosine substitutions within a CpG motif: cytosine to guanine at nucleotides 784, 1161, 1218, and 1966; cytosine to adenine at nucleotides 1264, 1337, 1829, 1874, 1940, and 1997; and cytosine to thymine at nucleotides 1158, 1963 and 1987; with additional cytosine to guanine mutations at nucleotides 1831, 1876, 1942, and 1999. (The nucleotide number designations as set forth above are according to the numbering system for pVAX1 provided by Invitrogen.)

The invention contemplates BHT-3021 vectors with added, deleted or substituted nucleotides that do not change the function of the BHT-3021 vector, *e.g.,* for expressing proinsulin and inhibiting an autoimmune response. Accordingly, the invention contemplates use of a self-vector comprising a polynucleotide encoding human proinsulin that shares at least about 95%, 97%, 98% or 99% nucleic acid sequence identity to SEQ ID NO:1, as measured using an algorithm known in the art, *e.g*., BLAST or ALIGN, set with standard parameters. Sequence identity can be determined with respect to, *e.g.,* the full-length of the BHT backbone, the full-length of the proinsulin autoantigen, or the full-length of the BHT-3021 vector.

Techniques for construction of vectors and transfection of cells are well-known in the art, and the skilled artisan will be familiar with the standard resource materials that describe specific conditions and procedures. The self-vector BHT-3021 is prepared and isolated using commonly available techniques for isolation of nucleic acids. The vector is purified free of bacterial endotoxin for delivery to humans as a therapeutic agent.

Modified self-vectors of this invention can be formulated as polynucleotide salts for use as pharmaceuticals. Polynucleotide salts can be prepared with non-toxic inorganic or organic bases. Inorganic base salts include sodium, potassium, zinc, calcium, aluminum, magnesium, etc. Organic non-toxic bases include salts of primary, secondary and tertiary amines, etc. Such self-DNA polynucleotide salts can be formulated in lyophilized form for reconstitution prior to delivery, such as sterile water or a salt solution. Alternatively, self-DNA polynucleotide salts can be formulated in solutions, suspensions, or emulsions involving water- or oil-based vehicles for delivery. In one embodiment, the DNA is lyophilized in phosphate buffered saline with physiologic levels of calcium (0.9 mM) and then reconstituted with sterile water prior to administration. Alternatively the DNA is formulated in solutions containing higher quantities of Ca⁺⁺, between 1 mM and 2M. The DNA can also be formulated in the absence of specific ion species.

### B. Compositions

Also described is a composition comprising a self-vector of SEQ ID NO:1 (BHT-3021). The composition can be formulated in a pharmaceutically acceptable carrier. In some cases, the pharmaceutical composition comprises calcium at a concentration about equal to physiological levels (e.g., about 0.9 mM). In some cases, the pharmaceutical composition further comprises a divalent cation at a concentration greater than physiological levels. In some cases, the divalent cation is calcium. In some cases, the self-vector is formulated with calcium at a concentration between about 0.9 mM (1x) to about 2 M; in some cases the calcium concentration is between about 2 mM to about 8.1 mM (9x); in some cases the calcium concentration is between about 2 mM to about 5.4 mM (6x). In some cases, the pharmaceutical composition is endotoxin-free.

In some cases, the self-vector is formulated with one or more divalent cations at a total concentration greater than physiological levels for injection into an animal for uptake by the host T cells of the animal. In some cases, one or more physiologically acceptable divalent cations can be used, *e.g.,* Ca²⁺, Mg²⁺, Mn²⁺, Zn²⁺, Al²⁺, Cu²⁺, Ni²⁺, Ba²⁺, Sr²⁺, or others, and mixtures thereof. In some cases, magnesium, calcium or mixtures thereof, can be present extracellularly at approximately 1.5 mM and 1 mM, respectively. Mixtures of two or more divalent cations can be used in combinations amounting to total concentrations of about 0.9, 2, 4, 5, 6, 7, 8, 9, 10, 12, 15, 20, 45, 65, 90, 130, 170, 220, 280, 320, 350, 500, 750, 1000, 1500 mM, etc., and up to about 2M.

In certain preferred case, the counterion can include PO₄, Cl, OH, CO₂, or mixtures thereof. In other cases, the formulations may cause DNA to form particulate or precipitates with size distributions where the mean sizes, or the 80% particles, are in excess of about 0.1, 0.3, 0.5, 1, 3, 5, 8, 15, 20, 35, 50, 70 or 100 microns. Size of such particulates may be evaluated by centrifugation, flow cytometry analysis, propydium iodide or similar dye labeling, or dynamic light scattering.

A pharmaceutical composition comprising BHT-3021 can be incorporated into a variety of formulations for therapeutic administration. More particularly, a combination as described herein can be formulated into pharmaceutical compositions, together or separately, by formulation with appropriate pharmaceutically acceptable carriers or diluents, and can be formulated into preparations in solid, semi-solid, liquid or gaseous forms, such as tablets, capsules, pills, powders, granules, dragees, gels, slurries, ointments, solutions, suppositories, injections, inhalants and aerosols. As such, administration of BHT-3021 can be achieved in various ways, including oral, buccal, parenteral, intravenous, intradermal, subcutaneous, intramuscular, transdermal, intrarectal, intravaginal, etc., administration. Moreover, the compound can be administered in a local rather than systemic manner, for example, in a depot or sustained release formulation.

Formulations suitable for use in the present invention are found in Remington: The Science and Practice of Pharmacy, 21st Ed., University of the Sciences in Philadelphia (USIP), Lippincott Williams & Wilkins (2005). The pharmaceutical compositions described herein can be manufactured in a manner that is known to those of skill in the art, i.e., by means of conventional mixing, dissolving, granulating, dragee-making, levigating, emulsifying, encapsulating, entrapping or lyophilizing processes. The following methods and excipients are merely exemplary and are in no way limiting.

In some embodiments, the self-vector can be formulated for intramuscular, subcutaneous, or parenteral administration by injection, *e.g*., by bolus injection or continuous infusion. For injection, BHT-3021 can be formulated into preparations by dissolving, suspending or emulsifying them in an aqueous or nonaqueous solvent, such as vegetable or other similar oils, synthetic aliphatic acid glycerides, esters of higher aliphatic acids or propylene glycol; and if desired, with conventional additives such as solubilizers, isotonic agents, suspending agents, emulsifying agents, stabilizers and preservatives. In some embodiments, the self-vector can be formulated in aqueous solutions, for example, in physiologically compatible buffers such as Hanks's solution, Ringer's solution, or physiological saline buffer. Formulations for injection can be presented in unit dosage form, e.g., in ampules or in multi-dose containers, with an added preservative. The compositions can take such forms as suspensions, solutions or emulsions in oily or aqueous vehicles, and can contain formulatory agents such as suspending, stabilizing and/or dispersing agents.

For oral administration, BHT-3021 can be readily formulated by combining the inhibitory agent with pharmaceutically acceptable carriers that are well known in the art. Such carriers enable the compounds to be formulated as tablets, pills, dragees, capsules, emulsions, lipophilic and hydrophilic suspensions, liquids, gels, syrups, slurries, suspensions and the like, for oral ingestion by a patient to be treated. Pharmaceutical preparations for oral use can be obtained by mixing the compounds with a solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents can be added, such as a crosslinked polyvinyl pyrrolidone, agar, or alginic acid or a salt thereof such as sodium alginate.

### C. Methods of Administration

The self-vector can be administered in a pharmaceutically acceptable carrier. In some embodiments, the self-vector BHT-3021 is administered in a pharmaceutically acceptable carrier or excipient comprising calcium at a concentration about equal to physiological levels (*e.g*., about 0.9 mM). In some embodiments, the self-vector BHT-3021 is administered in a pharmaceutically acceptable carrier or excipient comprising a divalent cation at a concentration greater than physiological levels. In some embodiments, the divalent cation is calcium. In some embodiments, the calcium is at a concentration greater than about 2 mM; in some embodiments, the calcium is at a concentration of about 5.4 mM. In some embodiments, the self-vector BHT-3021 is endotoxin-free. In some embodiments, the self-vector BHT-3021 is administered intramuscularly.

A wide variety of methods exist to deliver polynucleotide to subjects, as defined herein. For example, the polynucleotide encoding a self-polypeptide can be formulated with cationic polymers including cationic liposomes. Other liposomes also represent effective means to formulate and deliver self-polynucleotide. Alternatively, the DNA can be incorporated into a viral vector, viral particle, or bacterium for pharmacologic delivery. Viral vectors can be infection competent, attenuated (with mutations that reduce capacity to induce disease), or replication-deficient. Methods utilizing DNA to prevent the deposition, accumulation, or activity of pathogenic self proteins may be enhanced by use of viral vectors or other delivery systems that increase humoral responses against the encoded self-protein. In other embodiments, the DNA can be conjugated to solid supports including gold particles, polysaccharide-based supports, or other particles or beads that can be injected, inhaled, or delivered by particle bombardment (ballistic delivery). Methods for delivering nucleic acid preparations are known in the art.

A number of viral based systems have been developed for transfer into mammalian cells. For example, retroviral systems have been described. A number of adenovirus vectors have also been described. Adeno-associated virus (AAV) vector systems have also been developed for nucleic acid delivery. AAV vectors can be readily constructed using techniques well known in the art.

The polynucleotide of this invention can also be delivered without a viral vector. For example, the molecule can be packaged in liposomes prior to delivery to the subject. Lipid encapsulation is generally accomplished using liposomes which are able to stably bind or entrap and retain nucleic acid. For a review of the use of liposomes as carriers for delivery of nucleic acids.

Therapeutically effective amounts of self-vector are in the range of about 0.001 mg to about 1 g. A therapeutic amount of self-vector is in the range of about 10 ng to about 10 mg. For example, a therapeutic amount of self-vector is in the range of about 0.025 mg to 6 mg. In certain embodiments, the self-vector is administered monthly for 6-12 months, and then every 3-12 months as a maintenance dose. Alternative treatment regimens may be developed and may range from daily, to weekly, to every other month, to yearly, to a one-time administration depending upon the severity of the disease, the age of the patient, the self-polypeptide or -polypeptides being administered, and such other factors as would be considered by the ordinary treating physician.

In one embodiment, the polynucleotide is delivered by intramuscular injection. In other variations, the polynucleotide is delivered intranasally, orally, subcutaneously, intradermally, intravenously, mucosally, impressed through the skin, or attached to gold particles delivered to or through the dermis (*see, e.g.,* WO 97/46253). Alternatively, nucleic acid can be delivered into skin cells by topical application with or without liposomes or charged lipids (*see e.g.* U.S. Patent No. 6,087,341). Yet another alternative is to deliver the nucleic acid as an inhaled agent. The polynucleotide is formulated in phosphate buffered saline with physiologic levels of calcium (0.9 mM). Alternatively, the polynucleotide is formulated in solutions containing higher quantities of Ca⁺⁺, *e.g*., between 1 mM and 2M. The polynucleotide may be formulated with other cations such as zinc, aluminum, and others. Alternatively, or in addition, the polynucleotide may be formulated either with a cationic polymer, cationic liposome-forming compounds, or in non-cationic liposomes. Examples of cationic liposomes for DNA delivery include liposomes generated using 1,2-bis(oleoyloxy)-3-(trimethylammionio) propane (DOTAP) and other such molecules.

Prior to delivery of the polynucleotide, the delivery site can be preconditioned by treatment with bupivicane, cardiotoxin or another agent that may enhance the subsequent delivery of the polynucleotide. Such preconditioning regimens are generally delivered 12 to 96 hours prior to delivery of therapeutic polynucleotide; more frequently 24 to 48 hours prior to delivery of the therapeutic polynucleotide. Alternatively, no preconditioning treatment is given prior to polynucleotide therapy.

The self-vector can be administered in combination with other substances, such as, for example, pharmacological agents, adjuvants, cytokines, or vectors encoding cytokines. Furthermore, to avoid the possibility of eliciting unwanted anti-self cytokine responses when using cytokine codelivery, chemical immunomodulatory agents such as the active form of vitamin D3 can also be used. In this regard, 1,25-dihydroxy vitamin D3 has been shown to exert an adjuvant effect via intramuscular DNA immunization.

### EXAMPLES

In Type 1 diabetes there is an intense inflammatory response that destroys the beta cells in the islets of langerhans in the pancreas, the site were insulin is produced and released. Proinsulin is a major target of the adaptive immune response in Type 1 Diabetes (T1D). The present invention provides an engineered DNA plasmid encoding proinsulin (BHT-3021) that preserves beta cell function in T1D patients through reduction of insulin-specific CD8 T cells. We studied 80 subjects over 18 years of age who were diagnosed with T1D within the past five years. Subjects were randomized 2:1 to receive intramuscular injections of BHT 3021 or BHT-placebo, weekly for 12 weeks, then monitored for safety and immune responses in a blinded fashion. Four dose levels of BHT-3021 were evaluated: 0.3mg, 1.0mg, 3.0mg, and 6.0mg. C peptide was used both as an exploratory efficacy measure and as a safety measure. Islet-specific CD8 T cell frequencies were assessed with multimers of monomeric HLA Class I molecules loaded with peptides from pancreatic and unrelated antigens. No serious adverse events related to BHT-3021 were observed. C-peptide levels improved relative to placebo at all doses, at 1 mg at the 15 week time point (+19.5% BHT-3021 vs - 8.8% BHT-placebo, p<0.026). Proinsulin-reactive CD8 T cells, but not T cells against unrelated islet or foreign molecules, declined in the BHT-3021 arm (p<0.006). No significant changes were noted in Interferon-y, IL-4 or IL-10 production in CD4 T cells. Thus, we demonstrate that a plasmid encoding proinsulin reduces the frequency of CD8 T cells reactive to proinsulin, while preserving C-peptide over the course of dosing.

The following examples are offered to illustrate, but not to limit the claimed invention.

### METHODS

**Plasmid Construction.** BHT-3021 is a 3.3 Kb bacterial plasmid expression vector containing the coding sequences for the human proinsulin (hINS) gene. Important functional and control features of BHT-3021 were engineered into the final construct, including the human cytomegalovirus (CMV) immediate-early gene promoter/enhancer, the bovine growth hormone gene polyadenylation signal, the kanamycin resistance gene, and pUC origin of replication for propagation of the vector in *E. coli.* The backbone of BHT-3021 was modified to decrease the number of immunostimulatory CpG sequences. All nonessential CpG motifs were then substituted with immunomodulatory sequences, known as GpG sequences (*13*). Figure 1 shows the main structural features of BHT-3021. BHT-3021 was formulated in phosphate-buffered saline as a sterile solution for intramuscular (IM) injection, at a concentration of 2.0 mg DNA/mL. Placebo patients received phosphate-buffered saline.

**Enrollment and Recruitment.** The study was performed with informed consent from all subjects and under protocols that were approved by the Institutional Review Boards (IRBs) at each institution. Prior to initiating the clinical trial an Investigational New Drug Application (IND) was submitted to and accepted by the United States Food and Drug Administration (FDA) and approval from the NIH Recombinant DNA Advisory Committee was obtained. A total of 144 subjects were screened for the study. Eighty subjects (48 in the Dose Escalation cohorts and 32 subjects in the Expansion cohort) were randomized. **Inclusion criteria** were: 1) diagnosis of Type 1a Diabetes Mellitus based on American Diabetes Association (ADA) Criteria; 2) between 18 and 40 years of age; 3) within 5 years of diagnosis of T1D; 4) detectable fasting C-peptide; 5) C-peptide increase during mixed meal tolerance test (MITT) with a minimal stimulated value of ≥ 0.2 pmol/mL; 6) Presence of antibodies to at least one of the following antigens: insulin, GAD65 or IA2. If insulin antibody positive only, determination must have been completed within 2 weeks of insulin initiation; 7) agreement to intensive management of diabetes with a HbA_{1c} goal of < 7.0%; 8) if female, subjects must have been (a) surgically sterile, (b) postmenopausal or (c) if of reproductive potential, been willing to use medically acceptable birth control (e.g., female hormonal contraception, barrier methods or sterilization) until 3 months after completion of any treatment period; 9) if male and of reproductive potential, subjects must have been willing to use medically acceptable birth control until 3 months after completion of any treatment period, unless the female partner was postmenopausal or surgically sterile; and, 10) serum creatinine ≤ 1.5 x upper limit of normal (ULN); 11) AST < twice upper limit of normal; 12) white blood cells (WBC) ≥ 3 x 10⁹/L; platelets ≥ 100x 10⁹/L; and hemoglobin ≥ 10.0 g/dL. Subjects were excluded if 1) unable or unwilling to comply with the requirements of the study protocol; 2) Body Mass Index (BMI) > 30 kg/m²; 3) unstable blood sugar control, defined as one or more episodes of serious hypoglycemia (hypoglycemia that required the assistance of another person) within the 30 days prior to enrolment; 4) previous immune therapy for T1D; 5) administration of an experimental agent for T1D at any time, or use of an experimental device for T1D within 30 days prior to screening, unless approved by the Medical Monitor; 6) history of any organ transplant, including islet cell transplant; 7) active autoimmune or immune deficiency disorder other than T1D (e.g., sarcoidosis, rheumatoid arthritis), unless approved by the Medical Monitor; 8) 24-hour urinary albumin excretion > 300 mg at Screening; 9) uncontrolled or untreated retinopathy at screening; 10) serum bilirubin > ULN, except those subjects whose abnormal values were attributed to any stable, benign condition (such as Gilbert's Syndrome); 11) thyroid-stimulating hormone (TSH) outside the normal range at screening, except those subjects on stable doses of thyroid hormone replacement therapy; 12) known HIV positivity or evidence of high risk behavior; 13) Active hepatitis B or active hepatitis C infection; and 14) pregnant or lactating women.

**Trial Design.** The overall study design is shown in Figure 2. Subjects were screened for eligibility within 6 weeks prior to randomization. Subjects were randomized to BHT-3021 or BHT-placebo in a 2:1 ratio and entered the Blinded Treatment Period, when BHT-3021 or BHT-placebo was administered intramuscularly (IM) weekly for 12 weeks (Weeks 0 to 11). Four weeks after the last dose of BHT-3021 or BHT-placebo (Week 15), subjects underwent a complete evaluation for safety, beta cell function, and anti-insulin responses. Subjects were monitored for safety and immune response in a blinded fashion until 12 months after the first dose of BHT-3021 or BHT-placebo (the Blinded Evaluation Period). Each subject's treatment assignment was then unblinded. Subjects who received BHT-3021 entered a 12-month Long-Term Follow-Up (LTFU) Period, during which they were monitored for delayed adverse events, pancreatic function, and immune response. Subjects who received BHT-placebo were eligible for cross over to receive 12 weeks of treatment with BHT-3021 in an open-label manner.

Eighty subjects were enrolled in the study. Four dose levels of BHT-3021 were evaluated: 0.3 mg, 1.0 mg, 3.0 mg, and 6.0 mg. An initial 9 subjects were enrolled into an open-label cohort. After completion of the dose-finding phase of the study (Dose Escalation Phase), additional subjects were enrolled to expand one or more dose cohorts in order to obtain additional safety and efficacy data (Expansion Phase).

**Clinical Primary and Secondary Endpoints.** The Primary Objective was to evaluate the safety of BHT-3021 given as weekly injections over 12 weeks. The Secondary Objectives were to evaluate the effect of BHT-3021 on antibody and T-cell responses to diabetes-related antigens (insulin, GAD65, and IA2); to describe changes in pancreatic β-cell function after treatment with BHT-3021; and to describe changes in insulin requirements and blood glucose levels after treatment with BHT-3021.

**Primary Endpoints.** Subject Safety and Monitoring. The safety parameters assessed in the study were adverse events and serious adverse events; physical examinations; vital signs; clinical laboratory testing (hematology, chemistry, urinalysis); ophthalmologic examination; 12-lead ECG; 24-hour urine protein; stimulated C-peptide levels; pregnancy testing; and glucose measures (nighttime and self-monitored blood glucose).

**Secondary Endpoints.** C peptide was used both as an exploratory efficacy measure as well as a safety measure, to ensure that no dramatic decline in pancreatic function was observed with treatment with BHT-3021. Markers of metabolic control included HbA_{1c} and Fasting Plasma Glucose. Total daily insulin dose was assessed at baseline and during the study. The pharmacodynamic parameters assessed in the study were a) immune response to pancreatic antigens, as measured by antibodies to insulin, GAD65, and IA2, as well as T-cell responses to pancreatic antigens; and b) blood markers of immune activation.

**Antibodies to Pancreatic Antigens.** Radioimmunoassays were performed on baseline samples to determine the initial immune response to insulin. Analysis at subsequent time points was used to evaluate any change in the response that may have resulted from BHT-3021 treatment. Analysis of reactivity to GAD65 and IA2 was also measured as an overall indication of autoimmune responses to islet antigens. Antibodies to GAD65, IA2, and insulin (IAA) were measured at screening and were part of the entry criteria. Methods for detecting T1D-associated antibodies have been described previously (*3,26*).

Quantum Dot HLA-Peptide Multimers for Measurement of Frequency of Antigen Specific CD8 T cells. Multimeric HLA-A2-peptide complexes were prepared as previously described (*21*). Briefly, recombinant HLA-A2 and human β₂-microglobulin were solubilized in urea and injected together with each synthetic peptide into a refolding buffer consisting of 100 mmol/l Tris (pH 8.0), 400 mmol/l arginine, 2 mmol/l EDTA, 5 mmol/l reduced glutathione, and 0.5 mmol/l oxidized glutathione. Refolded complexes were biotinylated by incubation for 2 h at 30°C with BirA enzyme (Avidity, Denver, CO). The biotinylated complexes were purified by gel filtration on a Superdex 75 column (Amersham Pharmacia Biotech). Multimeric HLA-peptide complexes were produced by addition of streptavidin-conjugated quantum-dots (*21*) (Qdots; Invitrogen) to achieve a 1:20 streptavidin-Qdot/biotinylated HLA class I ratio. Qdot-585, -605, -655, -705, and -800 were used. Samples from HLA-A2/A3/B7 positive subjects were stained with a mixture containing nine diabetes-associated epitopes, an HLA-A2 epitope expressed in HLA-A2, and a mixture of viral antigens (table 6).

**Cell Staining with Qdot-Labeled Multimeric Complexes.** Peripheral blood mononuclear cells (PBMC) (2 × 10⁶) were stained simultaneously with all Qdot-labeled multimers (0.1 µg of each specific multimer) in 60 µl of PBS supplemented with 0.5% BSA and incubated for 15 min at 37°C. Subsequently, 10 µl allophycocyanin (APC)-labeled anti-CD8 (stock 1:10) and 10 µl fluorescein isothiocyanate (FITC)-labeled anti-CD4, -CD14, - CD16, -CD20, and -CD40 antibodies (Becton Dickinson) were added for 30 min at 4°C. After washing twice, cells were resuspended in PBS/0.5% BSA containing 7-aminoactinomycin D (7-AAD; eBioscience) to exclude dead cells, and analyzed using the LSR II (Becton Dickinson).

**Data Analysis and Statistical Methods.** Patients with HLA Class I type A2, A3 or B7 were stained with the corresponding multimers. Data were reported as the percentage of CD8 T cells that were specific for (or bound to) each multimer. Changes in antigen specific T cell percentages were calculated by subtracting the baseline values from each subsequent time point.

Analysis of islet specific immune response was performed by evaluating BHT-3021-specific responses separately from responses not specific to this agent. For example, for each patient the vaccine-specific changes were calculated for each appropriate multimer (Insulin B10-18, PPI 76-84 and PPI 79-88). The evaluation of islet specific non-vaccine responses were calculated for the peptides PPI 15-24, PPI 4-13, GAD65, IA2, IGRP and ppIAPP. In this case, a single patient could have as many as 6 different data points. For islet epitopes in the quantum dot combinatorial method specifically, the co-efficient of variation was determined at 10.8% (HLA-A2 peptide), 34.9% (virus mix); 15.9% (InsB), 0.0% (IA-2); 0.0% (IGRP), 6.3% (PPI), 4.5% (GAD65) and, 6.9% (ppIAPP)²¹.

Changes in CTL were calculated by subtracting the baseline values from values at week 15. Analysis was performed on all treated (all doses) and placebo patients positive for HLA-A2, -A3 and/or -B7. Statistics were performed using linear regression analysis.

ELISpot. ELISpots were performed on the first 48 patients enrolled in the dose escalation phase of the study. ELISpots were performed at the Barbara Davis Center for Childhood Diabetes (Aurora, CO). ELISpot data from these 48 patients are not presented due to low signal to noise ratio. The final 32 patients were included in the expansion phase and ELISpots were performed on these individuals at the CRO, Cellular Technologies Limited (CTL). PBMCs from patients from Australia/New Zealand were prepared at the CRO, Cancer Trials Australia, Melbourne. PBMCs from US patients were prepared at CTL. Frozen PBMCs were shipped in bulk from CTA to CTL where the assays were performed. IL-10 and IFN-gamma (IFN-γ) antigen specific immune responses were evaluated. The autoimmune response to insulin and GAD65 was measured as an indication of the ongoing autoimmune response to islet antigens. The immune response to a panel of viral peptides (CEF) was used to monitor irrelevant CD8 (not T1D associated) immune responses. The immune response to mosquito antigen was used to monitor antigen specific, but not diabetes related, CD4 T cell responses.

**Cross-Over Phase.** Subjects who received BHT-placebo were eligible for cross over to receive 12 weeks of treatment with BHT-3021 in an open-label manner. The dose of BHT-3021 during the Open Label Cross-Over Period was the "best dose" based on evaluation of available safety, immune response, and efficacy data. The "best dose" was defined as that dose or doses already administered in the clinical trial that the Data Safety Monitoring Board (DSMB) found to have an acceptable safety profile, and which the Sponsor determined at the time of cross over to present the best balance of safety, biological activity (immune response), and/or efficacy. More than one dose could have been designated as a "best dose," as long as all doses presented comparable safety and efficacy profiles. Crossover subjects were fully evaluated at the end of the dosing period (Week 15), after which they entered the Open Label Evaluation Period that lasted until 12 months after the first dose of BHT-3021. Finally, the subjects were entered in the 12-month long term followup period.

### RESULTS

Baseline Characteristics of the Intent to Treat Population. Table 1 shows that baseline characteristics of the Intent to Treat (ITT) population are not significantly different from those randomized to control.

### Pre-Specified Efficacy Endpoints.

**C-Peptide.** C-peptide secretion is considered an important surrogate marker for assessment of pancreatic secretion of insulin (*18-20*). Area under the curve of C-peptide response (referred to herein as "C-peptide") to mixed-meal tolerance test (MMTT) is a validated method of assessing endogenous insulin secretion, and Subjects with T1D have C-peptide responses to a MMTT at a time when intravenous glucose and glucagon responses were absent (*19,20*).

BHT-3021 (Fig 1) was dosed via the intramuscular route for 12 weeks, to individuals with type 1 diabetes who had residual C-peptide at the time of screening, (C-peptide> 33 pmol/L) (Fig. 2). Placebo received an equivalent dose of saline. At 15 weeks for 1.0 mg BHT-3021 dose versus BHT-placebo, the percent change from baseline in mean C-peptide was +19.5% (-1.95% lower confidence level, 41.0% upper confidence level, versus - 8.8% for placebo (-25.34% lower level of confidence, +7.66 upper confidence level), p<0.026 (Fig 3, fig. 5A). _Subjects in the 1.0 and 3.0 mg arms had C-peptide levels that were above the screening values at Week 15. Figure 5B shows percentage change from baseline for C-peptide in scatter plots of all doses and placebo at 15 weeks. In contrast, the placebo group, which started out higher, demonstrated a very steep decrease in C-peptide over the same 6-month period. One potential caveat was a longer mean time from diagnosis for the 1 mg group (59.7 months) compared to placebo (41.1 months), though this difference was not statistically significant (Table 1). These data suggest that BHT-3021 may preserve beta cell mass and/or function during the dosing period of 12 weeks and for up to 3 more months (6 month time point) after cessation of dosing. This effect is ultimately lost following discontinuation of therapy. Table 2 shows that treatment with BHT-3021 is not associated with a large reduction in C peptide.

Mean HbA_{1c}, Insulin Requirements and Blood Glucose Levels. HbA_{1c} allows a measure the changes in glucose homeostasis, over a long segment, as it reflects the glycosylation of hemoglobin, and thus reflects the status of plasma glucose, with the predominant contribution from plasma glucose over the past month. Generally levels of HbA_{1c} above 53 mmol/mol (7.0%) are considered diabetic, with standards varying depending on the organization who is deciding the guideline. Figure 6 displays the mean HbA_{1c} by treatment group for the MITT population. Differences in baseline HbA_{1C} among the groups were noted, reflecting varying levels of glycemic control at entry. The mean HbA_{1c} was relatively stable at entry and at 15 weeks, then increased after cessation of dosing at Month 6 in all groups, although differences were not statistically significant. Of note, there was a decrease in monitoring with fewer study visits beyond Week 15. Figure 6B displays the mean total insulin usage by treatment group. Total insulin usage was stable for the treatment groups for the initial 6 to 9 months of the study, and then increased subsequently. Mean insulin usage for the 1 mg dose fell during the period of dosing. The overall increase in insulin usage was concurrent with higher HbA_{1C}. In particular, over the duration of study drug dosing, insulin usage was stable when compared to baseline in each of the treatment groups, though the differences were not statistically significant from placebo.

### Immunological Studies

Enumeration of Antigen Specific CD8 T Cells During Therapy. A pre-specified immunological study was designed to quantify the changes in islet-specific CD8 T cells before and after treatment with BHT-3021. All patients were typed for HLA. Sixty four patients had HLA Class I types for which multimers were available. Twenty one of the 64 patients had too few cells at baseline to allow comparison over time. Two patient samples were not collected. Therefore, a total of 41 of the 80 patients were evaluated at baseline and at least one time point following treatment. We used the combinatorial quantum dot (Qdot) technique (*21*) to simultaneously detect CD8+ T-cells specific for nine different beta-cell-derived antigens, and a cadre of viral epitopes, to measure responses to non-islet antigens (*21*).

We analyzed delta (stimulated) C-peptide in relation to changes in CD8 islet autoreactivity from baseline in patients treated with active drug compared to placebo, for each of the epitopes tested, and for HLA-A2, HLA-A3 and HLA-B7. We then distinguished epitopes present in the BHT DNA vaccine (i.e. proinsulin, but not the leader peptide in PPI) from other islet autoantigens (GAD, IA2, PPI leader sequence, IAPP, and IGRP). Finally, we accounted for one insulin epitope (ins B10-18), which is also present in injected insulin, which was used for insulin replacement therapy in all patients in the study (*22*). Since it is known that immune responses to injected insulin may develop after initiation of insulin therapy, insulin replacement may act as confounder regarding changes induced by BHT-3021. Therefore CD8 T-cell responses to this epitope were separated from the two other epitopes present in BHT-3021. Finally, we distinguished no change in T-cell response (delta=0) in cases where there was no response detectable at any time reliably, from those where the frequencies were the same at t=0 and t=15 weeks.

When the change in the frequency of CD8 lymphocytes to proinsulin was compared with the percent change in the mean C-peptide, there was a negative correlation for proinsulin, but not for insulin or other beta cell antigens including preproinsulin, IA2, IGRP, GAD65 or ppIAPP, (Fig. 4, p=0.006 for HLA-A3 Proinsulin, treated vs. placebo, using linear regression analysis, n= 12 and n= 8 respectively; p>0.05 for all other epitopes). These results indicate that BHT-3021 induced antigen-specific reductions in CD8 cells reactive to proinsulin, but not to other antigens, and that the magnitude of the reduction was inversely correlated with the improvement in C-peptide.

Analysis of the frequencies of virus-specific CD8 T cells over time showed no differences between treated subjects vs. placebo. CTL frequencies against vaccine epitopes significantly increased in placebo over time, compared to treated patients (p=0.003 using one-tailed Mann Whitney test at week 15, (n= 16 for placebo and n= 30 for treated, see fig. 7A). For proinsulin HLA-A3-specific CD8 T cells, differences in placebo versus treated were most pronounced at week 15, with differences waning after cessation of therapy (n= 8 for placebo and n= 16 for treatedp=0.0142 using Mann Whitney test at week 15, fig. 7B).

Treatment arms were evenly distributed for the criterion of baseline CD8 islet autoreactivity, ruling out the possibility that the changes in T-cell response to BHT-3021 at 15 weeks were due to selective imbalance seen at time 0 (Figure 8).

**ELISPOT Analysis of Cytokine Production in CD4 T Cells Specific for Insulin B9-23 and Other Islet Cell Antigens.** We chose to measure IFN-γ and IL-10, because IFN-γ is the major Th1 cytokine and IL-10 is a key cytokine produced by regulatory T cells. There was no consistent change in IL-10 immune responses to any islet epitopes including those contained in BHT-3021 as well as those unrelated to BHT-3021 (Figure 9A), and no change in IFN-γ responses to the immunodominant insulin epitope (Figuire 9B), at 15 weeks. There were insufficient data available for correlation of ELISPOT analysis and CD8 multimer analysis for the same patient at matching time points.

**Autoantibodies to pancreatic antigens.** Autoantibodies to pancreatic antigens were measured at baseline and Week 15 (3 weeks post final BHT-3021 administration) in all subjects for which samples were available. In general, there were few changes in antibody status at Week 15 such that individuals who were positive at baseline for a specific antibody maintained positivity at Week 15, and, conversely if they were negative at baseline, they remained negative at Week 15 (table S2). A few exceptions existed, specifically a single placebo subject who converted from negative to positive for GAD65, and four subjects (2 active and 2 placebo) converted from negative to positive for insulin antibodies (IA). No subjects converted from negative to positive for IA2.

Since the plasmid DNA BHT-3021 encodes the proinsulin protein, changes in the immune response to insulin were of particular interest. In order to determine if the change in IA status in these 4 subjects correlated with any clinical outcomes, the changes in C peptide at Week 5, Week 15, and Month 6 for the subjects converting from negative to positive for IA confirmed that there were no consistent C-peptide changes that correlated with the induction of insulin antibodies. Interestingly, the subject with the largest induction of IA had the best preservation of C peptide over time. We conclude that the induction of IA did not correlate with an undesirable precipitous decline in C peptide in these subjects.

We also saw improvement in Hemoglobin A1c (HgbA1c) relative to placebo, when all doses of the drug were pooled vs. placebo, and monitored at the 11 week time point and compared to time zero (dosing was for 12 weeks). Hemoglobin A1c provides an average of blood sugar control over a six to 12 week period. There was a decline in HgbAlc -0.22% compared to an increase of 0.0045% in placebo (Table 5).

### Safety

**Treatment Emergent Adverse Events (TEAEs).** The independent Data Saftey Monitoring Board determined that there were no treatment related adverse events appeared to be related to the study drug. Detailed description of all TEAEs are presented in Table 4. Summary statistics consisted of numbers and percentages of subjects for categorical measures and means, medians, standard deviations, and minimum and maximum values for continuous measures as calculated with Version 9.1.3 of the SAS® statistical software package for the calculation of all summaries, listings, graphs, and statistical analyses of adverse events.

TEAEs were reported for 12 (85.7%) of 14 subjects treated with 0.3 mg BHT-3021; 18 (100%) of 18 subjects treated with 1.0 mg; 11 (78.6%) of 14 subjects treated with 3.0 mg; 7 (87.5%) of 8 subjects treated with 6.0 mg; and 25 (96.2%) of 26 subjects treated with BHT-Placebo. Grade 3 or higher TEAEs were reported for 4 (28.6%) of 14 subjects treated with 1.0 mg BHT-3021; 2 (28.6%) of 14 subjects treated with 3.0 mg; 3 (37.5%) of 8 subjects treated with 6.0 mg; and 4 (15.4%) of 26 subjects treated with BHT-Placebo. The various types of TEAE's, none related to the study drug, are summarized in Table 4. TEAEs considered to be possibly related to study drug were reported for 5 (35.7%) of 14 subjects treated with 0.3 mg BHT-3021; 6 (33.3%) of 18 subjects treated with 1.0 mg; 6 (42.9%) of 14 subjects treated with 3.0 mg; 4 (50.0%) of 8 subjects treated with 6.0 mg; and 6 (23.1%) of 26 subjects treated with BHT-Placebo. Most of these events were noted by the Investigator to be Grade 1; a few were Grade 2 events. Serious TEAEs were reported for 1 (7.1%) of 14 subjects treated with 0.3 mg BHT-3021 for 1 (5.6%) of 18 subjects treated with 1.0 mg, and 4 (15.4%) of 26 subjects treated with BHT-Placebo; none of these events were considered to be related to study drug.

**Discontinuations.** Two subjects treated with 3.0 mg BHT-3021 were discontinued from study drug treatment due to TEAEs that investigators could not be certain were unrelated to study drug. One subject reported a Grade 2 headache, and one subject developed Grade 1 vaginal candidiasis. Upon completion of the trial and review of data on all patients, there was no statistical association of these particular adverse events, or any others, to study drug. There were no deaths in the study. We conclude that BHT-3021 met its primary endpoint for safety, with no substantial toxicities noted.

### DISCUSSION

There is no approved immunotherapy for the treatment of T1D. The mainstay of treatment is insulin replacement, a lifesaving breakthrough that was discovered more than 90 years ago. A therapeutic agent that targets the primary pathogenesis of the disease has long been sought.

A major autoimmune response in T1D is directed to insulin (*1-3,5,6*). In this study we have attempted to modulate, in an antigen-specific manner, the adaptive immune response to proinsulin with an engineered DNA vaccine encoding proinsulin. The vaccine is engineered to reduce the immunogenicity of the encoded proinsulin by substituting CpG hexameric motifs, which stimulate the innate immune response, with GpG hexameric nucleotide sequences, known to modulate innate immunity (*13*). Here we show that this approach modulated C-peptide, with an actual rise in this marker of beta cell function during the dosing period at two doses. We also demonstrate that as the C-peptide increases there is a deletion of CD8 T cells reactive to proinsulin, but there is no effect on other antigen specific T cell responses. This is a firm indication that antigen specific modulation has occurred.

There was no increase in adverse events or in serious adverse events associated with BHT-3021 (table 4). This is a particularly important outcome, since T1D is more commonly observed in children and young adults where BHT-3021 will ultimately need to be tested.

We assessed C-peptide to ascertain whether this vaccine might cause an undesirable precipitous fall in C-peptide. To the contrary, we observed significant improvement in C-peptide during the dosing period. The 1 mg dose was most effective compared to placebo, p<0.026 (Fig. 3). Treatment with 1.0 and 3.0 of BHT-3021 led to C-peptide levels that were above the screening values at Week 15. Thus, these data provide evidence of preservation of C-peptide during the dosing period, an effect that was lost when subjects were no longer exposed to the antigen-encoding vaccine. This result is surprising and unexpected, since the trial was not powered to measure efficacy outcomes, and because the trial was performed in adults with disease duration up to 5 years, and proportionately lower beta cell mass and perhaps more end-stage immune responses, than those observed in recent onset diabetic subjects.

HgbA_{1c} was well controlled during dosing of the DNA plasmid compared to placebo (fig. 6A). The mean HbA_{1c} was relatively stable initially through 15 weeks of treatment with BHT-3021, then increased at month 6 in all groups. Insulin usage appeared relatively stable overall when compared to baseline in each of the treatment groups (fig. 6B). The data from week 104 week are not statistically significant, N=3.
Neither the HbA_{1c} nor the insulin usage data were significantly different from control at any dose.

CD8 T cells are critical in the pathogenesis of T1D (*1-3,5,6*). CD8 T cells specific for proinsulin, other islet cell antigens, and viral antigens were assessed with HLA class I multimers, a technology that allows for enumeration of the frequency of antigen-specific T cells with flow cytometry (*21,23*). We demonstrate antigen specific reduction in CD8 cells reactive to proinsulin, but not to other antigens, and that the magnitude of the reduction was inversely correlated with the improvement in C-peptide (Fig. 4).

CD8 T cells specific for proinsulin have been detected in the islets of patients with T1D employing the same HLA monomers used in our studies (*1*). Reduction in the frequency of such CD8 T cells in this study correlated with increases in C-peptide during the period of dosing (s1 4). We speculate that proinsulin-specific CD8 T cells are either deleted by apoptosis because they receive signals through their cognate T cell receptors in the absence of costimulatory signals provided by antigen presenting cells, or that they are actively suppressed by regulatory T cells and sequestered from the pancreatic islets, and from the peripheral circulation where we attempted to detect them.

The particular HLA types and epitopes used in the analysis with multimers are relevant to the pathophysiology of T1D. A recent study using tetramers, rather than the quantum dot multimers employed in the current paper, but with the same HLA molecules and islet epitopes as the ones used in the current experiments for BHT3021, detected similar CD8 T cells in peripheral blood, that are also seen in the inflamed pancreas of the same patient with T1D (*24*). Thus these peripheral CD8 T cells found in the circulation are known to locate in the inflamed islets (*24*). Another recent investigation revealed that CD8 T-cells cloned from peripheral blood and reactive against one of the epitopes in the multimer study used in this paper were pathogenic (*25*). These CD8 clones caused insulitis and beta-cell destruction when injected into humanized (HLA-A2 transgenic) mice, demonstrating diabetogenicity of these particular circulating islet autoreactive human CD8 T-cells detected in our assay in this clinical trial (*25*). These T cells under investigation in this clinical trial may thus have real pathogenic relevance to T1D (*24,25*).

Taken together, the preservation of C-peptide during the period of dosing of BHT-3021 along with the immunological studies with MHC class I multimers indicate that BHT-3021 induces antigen specific modulation of the immune response to proinsulin, but not to other antigens. A long sought after goal of therapy in autoimmune disease aims to reduce or abolish the unwanted autoimmune responses that contribute to pathology. There is strong evidence that immunity to insulin, a primary beta cell specific antigen, is one of the fundamental aspects underlying the pathophysiology of T1D. The results of this twelve week trial with an engineered DNA plasmid encoding proinsulin indicate that there is antigen specific suppression of immunity to proinsulin, during the period of dosing. Longer trials with BHT-3021 are warranted, given the reduction in immunity to proinsulin and the favorable safety profile.

### TABLES

**Table 1. Demographics and Baseline Characteristics (ITT Population)**

| | **0.3 mg (n=14)** | **1.0 mg (n=18)** | **3.0 mg (n=14)** | **6.0 mg (n=8)** | **Placebo (n=26)** |
|---|---|---|---|---|---|
| Mean Age (years) | 29.6 | 31.5 | 31.8 | 27.6 | 29.3 |
| Gender | | | | | |
| Male | 9 (64.3%) | 10 (55.6%) | 6 (42.9%) | 7 (87.5%) | 18 (69.2%) |
| Female | 5 (35.7%) | 8 (44.4%) | 8 (57.1%) | 1 (12.5%) | 8 (30.8%) |
| Race | | | | | |
| Caucasian | 10 (71.4%) | 17 (94.4%) | 11 (78.6%) | 8 (100%) | 22 (84.6%) |
| Asian | 1 (7.1%) | 0 | 1 (7.1%) | 0 | 1 (3.8%) |
| Black | 0 | 1 (5.6%) | 1 (7.1%) | 0 | 1 (3.8%) |
| Hispanic | 2 (14.3%) | 0 | 0 | 0 | 1 (3.8%) |
| American Indian or Alaska Native | 0 | 0 | 1 (7.1%) | 0 | 0 |
| Other | 1 (7.1%) | 0 | 0 | 0 | 1 (3.8%) |
| Mean time from diagnosis (months) | 14.0 | 59.7 | 36.9 | 32.2 | 41.1 |

| **Table 2: Incidence of Precipitous Decline* in C Peptide** | | | | | | |
|---|---|---|---|---|---|---|
| **Timepoint** | **0.3 mg (n=14)** | **1.0 mg (n=15)** | **3.0 mg (n=12)** | **6.0 mg (n=8)** | **Pooled Active (n=49)** | **Placebo (n=23)** |
| Month 6 | | | | | | |
| Decrease ≥75%, n (%) | 0 | 1 (6.7%) | 0 | 0 | 1 (2.0%) | 0 |
| Month 12 | | | | | | |
| Decrease ≥75%, n (%) | 1 (7.1%) | 1 (6.7%) | 1 (8.3%) | 0 | 3 (6.1%) | 3 (13%) |

| | | | | | | |
|---|---|---|---|---|---|---|
| * Decrease ≥75% in C peptide | | | | | | |

| **Table 3. Change in Antibody Status at Week 15** | | |
|---|---|---|
| **Dose Levels** | **GAD65¹** | **IAA²** |
| **(mg)** | **NEG to POS** | **NEG to POS** |
| 0.3 | 0/14 | 1/14 (Subject 42-005) |
| 1 | 0/15 | 0/15 |
| 3 | 0/13 | 1/13 (Subject 42-003) |
| 6 | 0/8 | 0/8 |
| 0 (placebo) | 1/23 (42-004) | 2/23 (Subjects 31-007 and 42-004) |

| | | |
|---|---|---|
| ¹GAD65- measured by RIA using index value of >0.032 as positive cut-off ²IAA - measured by RIA using index value of >0.01 as positive cut-off | | |

| **Table 4. Treatment Emergent Adverse Events by Treatment Group (ITT Population)** | | | | | |
|---|---|---|---|---|---|
| **MedDRA Body System** | **0.3 mg n=14 n (%)** | **1.0 mg n=18 n (%)** | **3.0 mg n=14 n (%)** | **6.0 mg n=8 n (%)** | **Placebo n=26 n (%)** |
| Subjects with Any AE | 12 (85.7%) | 18 (100%) | 11 (78.6%) | 7 (87.5%) | 25 (96.2%) |
| Blood and Lymphatic System Disorders | 1 (7.1%) | | 3 (21.4%) | | 1 (3.8%) |
| Cardiac Disorders | | | | 1 (12.5%) | 1 (3.8%) |
| Ear and Labyrinth Disorders | | | 1 (7.1%) | | |
| Eye Disorders | 1 (7.1%) | 2 (11.1%) | 1 (7.1%) | | 2 (7.7%) |
| Gastrointestinal Disorders | 2 (14.3%) | 6 (33.3%) | 5 (35.7%) | 1 (12.5%) | 6 (23.1%) |
| General Disorders and Administration Site Conditions | 2 (14.3%) | 1 (5.6%) | 1 (7.1%) | 3 (37.5%) | 1 (3.8%) |
| Immune System Disorders | 1 (7.1%) | | | | 4 (15.4%) |
| Infections And Infestations | 10 (71.4%) | 13 (72.2%) | 9 (64.3%) | 6 (75.0%) | 13 (50.0%) |
| Injury, Poisoning and Procedural Complications | 3 (21.4%) | 3 (16.7%) | 3 (21.4%) | 2 (25.0%) | 4 (15.4%) |
| Metabolism and Nutrition Disorders | 3 (21.4%) | 2 (11.1%) | 3 (21.4%) | 3 (37.5%) | 12 (46.2 %) |
| Musculoskeletal and Connective Tissue Disorders | 2 (14.3%) | 1 (5.6%) | 4 (28.6%) | 1 (12.5%) | 3 (11.5%) |
| Nervous System Disorders | 4 (28.6%) | 6 (33.3%) | 5 (35.7%) | 2 (25.0%) | 8 (30.8%) |
| Psychiatric Disorders | | | 2 (14.3%) | | 1 (3.8%) |
| Renal and Urinary Disorders | 1 (7.1%) | 1 (5.6%) | | | |
| Respiratory, Thoracic and Mediastinal Disorders | 1 (7.1%) | 3 (16.7%) | 4 (28.6%) | 1 (12.5%) | 8 (30.8%) |
| Skin and Subcutaneous Tissue Disorders | 3 (21.4%) | 4 (22.2%) | 3 (21.4%) | 2 (25.0%) | 8 (30.8%) |
| Surgical and Medical Procedures | | 1 (5.6%) | | | 1 (3.8%) |
| Metabolism and Nutrition Disorders | 3 (21.4%) | 2 (11.1%) | 3 (21.4%) | 3 (37.5%) | 12 (46.2 %) |
| Musculoskeletal and Connective Tissue Disorders | 2 (14.3%) | 1 (5.6%) | 4 (28.6%) | 1 (12.5%) | 3 (11.5%) |
| Nervous System Disorders | 4 (28.6%) | 6 (33.3%) | 5 (35.7%) | 2 (25.0%) | 8 (30.8%) |
| Psychiatric Disorders | | | 2 (14.3%) | | 1 (3.8%) |
| Renal and Urinary Disorders | 1 (7.1%) | 1 (5.6%) | | | |
| Respiratory, Thoracic and Mediastinal Disorders | 1 (7.1%) | 3 (16.7%) | 4 (28.6%) | 1 (12.5%) | 8 (30.8%) |
| Skin and Subcutaneous Tissue Disorders | 3 (21.4%) | 4 (22.2%) | 3 (21.4%) | 2 (25.0%) | 8 (30.8%) |
| Surgical and Medical Procedures | | 1 (5.6%) | | | 1 (3.8%) |

| **Table 5. Combinations of Q-dot labeled HLA-A2, A3 and B7 multimers.** | | | | | | |
|---|---|---|---|---|---|---|
| **Origin** | **Islet specific** | **Insulin Specific** | **Position/protein** | **Sequence** | **HLA Restriction** | **Signal** |
| CMV | no | NA | pp65 | NLVPMVATV | HLA-A2 | Qdot 585 + 655 |
| EBV | no | NA | LMP2 | CLGGLLTMV | HLA-A2 | Qdot 585 + 655 |
| Measles | no | NA | H250 | SMYRVFEVGV | HLA-A2 | Qdot 585 + 655 |
| HLA-A2 | no | NA | 140-149 | YAYDGKDYIA | HLA-A2 | Qdot 585 + 605 |
| Insulin | yes | yes | B 10-18 | HLVEALYLV | HLA-A2 | Qdot 605 + 655 |
| PPI | yes | yes | 15-24 | ALWGPDPAAA | HLA-A2 | Qdot 705 + 655 |
| GAD65 | yes | no | 114-123 | VMNiLLQYVV | HLA-A2 | Qdot 800 + 655 |
| IA-2 | yes | no | 797-805 | MVWESGCTV | HLA-A2 | Qdot 705 + 605 |
| IGRP | yes | no | 265-273 | VLFGLGFAI | HLA-A2 | Qdot 800 + 605 |
| ppIAPP | yes | no | 5-13 | KLQVFLIVL | HLA-A2 | Qdot 705 + 800 |
| PPI₇₆₋₈₄ | yes | yes | 76-84 | SLQPLALEG | HLA-A3 | Qdot 585 + 800 |
| PPI₇₉₋₈₈ | yes | yes | 79-88 | PLALEGSLQK | HLA-A3 | Qdot 585 + 705 |
| PPI₄₋₁₃ | yes | yes | 4-13 | WMRLLPLLAL | HLA-B7 | Qdot 585 + Qdot 800 or Qdot 655 + 705* |
| CMV, cytomegalovirus; EBV, Epstein-Barr virus. | | | | | | |
| *: depending on the mix with either HLA-A3 or HLA-A2 epitopes combined. | | | | | | |

**Table 6**

| TOL-3021 | | | | Page 1 of 1 | | | | |
|---|---|---|---|---|---|---|---|---|
| **Table 14.2.43.3** | | | | | | | | |
| **Paired T-test for HbA1c (%) at Baseline vs Week 11 (All Doses vs Placebo)** | | | | | | | | |
| **ITT Population with Baseline C-Peptide > 33 pmol/L** | | | | | | | | |
| _**Dose** | **N** | **Mean (LCL, UCL)** | **Standard Deviation** | | **Standard Error** | **t-value** | **DF** | **p-value** |
| | | | | | | | | |
| _All Doses | 50 | -0.2200 (-0.4708, 0.2429) | 0.8825 | | 0.1248 | -1.76 | 49 | 0.0842 |
| _Placebo | 22 | 0.0045 (-0.2338, 0.2429) | 0.5376 | | 0.1146 | 0.04 | 21 | 0.9687 |

### REFERENCES and Notes

1. K.T. Coppieters, F. Dotta, N. Amirian, P.D. Campbell, T.W. Kay, M.A. Atkinson, B.O. Roep & M.G. von Herrath, Demonstration of islet-autoreactive CD8 T cells in insulitic lesions from recent onset and long-term type 1 diabetes patients. J Exp Med. 209, 51-60 (2012).
2. G.G. Pinkse, O.H. Tysma, C.A. Bergen, M.G. Kester, F. Ossendorp, P.A. van Veelen, B. Keymeulen, D. Pipeleers, J.W. Drijfhout & B.O. Roep, Autoreactive CD8 T cells associated with beta cell destruction in type 1 diabetes. Proc Natl Acad Sci U S A. 102, 18425-18430 (2005).
3. C.F. Verge, D. Stenger, E. Bonifacio, P.G. Colman, C. Pilcher, P.J. Bingley & G.S. Eisenbarth, Combined use of autoantibodies (IA-2 autoantibody, GAD autoantibody, insulin autoantibody, cytoplasmic islet cell antibodies) in type 1 diabetes: Combinatorial Islet Autoantibody Workshop. Diabetes. 47, 1857-1866 (1998).
4. A. Kupila, J. Sipila, P. Keskinen, T. Simell, M. Knip, K. Pulkki & O. Simell, Intranasally administered insulin intended for prevention of type 1 diabetes--a safety study in healthy adults. Diabetes Metab Res Rev. 19, 415-420 (2003).
5. J.S. Skyler, J.P. Krischer, J. Wolfsdorf, C. Cowie, J.P. Palmer, C. Greenbaum, D. Cuthbertson, L.E. Rafkin-Mervis, H.P. Chase & E. Leschek, Effects of oral insulin in relatives of patients with type 1 diabetes: The Diabetes Prevention Trial--Type 1. Diabetes Care. 28, 1068-1076 (2005).
6. J.S. Skyler, R.S. Weinstock, P. Raskin, J.F. Yale, E. Barrett, J.E. Gerich & H.C. Gerstein, Use of inhaled insulin in a basal/bolus insulin regimen in type 1 diabetic subjects: a 6-month, randomized, comparative trial. Diabetes Care. 28, 1630-1635 (2005).
7. A.A. Mamchak, Y. Manenkova, W. Leconet, Y. Zheng, J.R. Chan, C.L. Stokes, L.K. Shoda, M. von Herrath & D. Bresson, Preexisting autoantibodies predict efficacy of oral insulin to cure autoimmune diabetes in combination with anti-CD3. Diabetes. 61, 1490-1499 (2012).
8. J. Ludvigsson, D. Krisky, R. Casas, T. Battelino, L. Castano, J. Greening, O. Kordonouri, T. Otonkoski, P. Pozzilli, J.J. Robert, H.J. Veeze, J. Palmer, U. Samuelsson, H. Elding Larsson, J. Aman, G. Kardell, J. Neiderud Helsingborg, G. Lundstrom, E. Albinsson, A. Carlsson, M. Nordvall, H. Fors, C.G. Arvidsson, S. Edvardson, R. Hanas, K. Larsson, B. Rathsman, H. Forsgren, H. Desaix, G. Forsander, N.O. Nilsson, C.G. Akesson, P. Keskinen, R. Veijola, T. Talvitie, K. Raile, T. Kapellen, W. Burger, A. Neu, I. Engelsberger, B. Heidtmann, S. Bechtold, D. Leslie, F. Chiarelli, A. Cicognani, G. Chiumello, F. Cerutti, G.V. Zuccotti, A. Gomez Gila, I. Rica, R. Barrio, M. Clemente, M.J. Lopez Garcia, M. Rodriguez, I. Gonzalez, J.P. Lopez, M. Oyarzabal, H.M. Reeser, R. Nuboer, P. Stouthart, N. Bratina, N. Bratanic, M. de Kerdanet, J. Weill, N. Ser, P. Barat, A.M. Bertrand, J.C. Carel, R. Reynaud, R. Coutant & S. Baron, GAD65 antigen therapy in recently diagnosed type 1 diabetes mellitus. N Engl J Med. 366, 433-442 (2012).
9. http://www.andromedabio.com/page.php?pageID=80, accessed April 28, 2013
10. D. Bresson & M. von Herrath, Humanizing animal models: a key to autoimmune diabetes treatment. Sci Transl Med. 3, 68ps64 (2011).
11. B. Coon, L.L. An, J.L. Whitton & M.G. von Herrath, DNA immunization to prevent autoimmune diabetes. J Clin Invest. 104, 189-194 (1999).
12. N. Solvason, Y.P. Lou, W. Peters, E. Evans, J. Martinez, U. Ramirez, A. Ocampo, R. Yun, S. Ahmad, E. Liu, L. Yu, G. Eisenbarth, M. Leviten, L. Steinman & H. Garren, Improved efficacy of a tolerizing DNA vaccine for reversal of hyperglycemia through enhancement of gene expression and localization to intracellular sites. J Immunol. 181, 8298-8307 (2008).
13. P.P. Ho, P. Fontoura, P.J. Ruiz, L. Steinman & H. Garren, An immunomodulatory GpG oligonucleotide for the treatment of autoimmunity via the innate and adaptive immune systems. J Immunol. 171, 4920-4926 (2003).
14. B.O. Roep, The role of T-cells in the pathogenesis of Type 1 diabetes: from cause to cure. Diabetologia. 46, 305-321 (2003).
15. A.J. Williams, A.J. Norcross, R.J. Dix, K.M. Gillespie, E.A. Gale & P.J. Bingley, The prevalence of insulin autoantibodies at the onset of Type 1 diabetes is higher in males than females during adolescence. Diabetologia. 46, 1354-1356 (2003).
16. S.C. Kent, Y. Chen, L. Bregoli, S.M. Clemmings, N.S. Kenyon, C. Ricordi, B.J. Hering & D.A. Hafler, Expanded T cells from pancreatic lymph nodes of type 1 diabetic subjects recognize an insulin epitope. Nature. 435, 224-228 (2005).
17. S. Arif, T.I. Tree, T.P. Astill, J.M. Tremble, A.J. Bishop, C.M. Dayan, B.O. Roep & M. Peakman, Autoreactive T cell responses show proinflammatory polarization in diabetes but a regulatory phenotype in health. J Clin Invest. 113, 451-463 (2004).
18. J.P. Palmer, G.A. Fleming, C.J. Greenbaum, K.C. Herold, L.D. Jansa, H. Kolb, J.M. Lachin, K.S. Polonsky, P. Pozzilli, J.S. Skyler & M.W. Steffes, C-peptide is the appropriate outcome measure for type 1 diabetes clinical trials to preserve beta-cell function: report of an ADA workshop, 21-22 October 2001. Diabetes. 53, 250-264 (2004).
19. C.J. Greenbaum & L.C. Harrison, Guidelines for intervention trials in subjects with newly diagnosed type 1 diabetes. Diabetes. 52, 1059-1065 (2003).
20. C.J. Greenbaum, T. Mandrup-Poulsen, P.F. McGee, T. Battelino, B. Haastert, J. Ludvigsson, P. Pozzilli, J.M. Lachin & H. Kolb, Mixed-meal tolerance test versus glucagon stimulation test for the assessment of beta-cell function in therapeutic trials in type 1 diabetes. Diabetes Care. 31, 1966-1971 (2008).
21. J.H. Velthuis, W.W. Unger, J.R. Abreu, G. Duinkerken, K. Franken, M. Peakman, A.H. Bakker, S. Reker-Hadrup, B. Keymeulen, J.W. Drijfhout, T.N. Schumacher & B.O. Roep, Simultaneous detection of circulating autoreactive CD8+ T-cells specific for different islet cell-associated epitopes using combinatorial MHC multimers. Diabetes. 59, 1721-1730 (2010).
22. N.C. Schloot, B.O. Roep, D. Wegmann, L. Yu, H.P. Chase, T. Wang & G.S. Eisenbarth, Altered immune response to insulin in newly diagnosed compared to insulin-treated diabetic patients and healthy control subjects. Diabetologia. 40, 564-572 (1997).
23. J.D. Altman, P.A. Moss, P.J. Goulder, D.H. Barouch, M.G. McHeyzer-Williams, J.I. Bell, A.J. McMichael & M.M. Davis, Phenotypic analysis of antigen-specific T lymphocytes. Science. 274, 94-96 (1996).
24. J.H. Velthuis, W.W. Unger, A.R. van der Slik, G. Duinkerken, M. Engelse, A.F. Schaapherder, J. Ringers, C. van Kooten, E.J. de Koning & B.O. Roep, Accumulation of autoreactive effector T cells and allo-specific regulatory T cells in the pancreas allograft of a type 1 diabetic recipient. Diabetologia. 52, 494-503 (2009).
25. W.W. Unger, T. Pearson, J.R. Abreu, S. Laban, A.R. van der Slik, S.M. der Kracht, M.G. Kester, D.V. Serreze, L.D. Shultz, M. Griffioen, J.W. Drijfhout, D.L. Greiner & B.O. Roep, Islet-specific CTL cloned from a type 1 diabetes patient cause beta-cell destruction after engraftment into HLA-A2 transgenic NOD/scid/IL2RG null mice. PloS one. 7, e49213 (2012).
26. L. Yu, D.T. Robles, N. Abiru, P. Kaur, M. Rewers, K. Kelemen & G.S. Eisenbarth, Early expression of antiinsulin autoantibodies of humans and the NOD mouse: evidence for early determination of subsequent diabetes. Proc Natl Acad Sci U S A. 97, 1701-1706 (2000).

### SEQUENCE LISTING

SEQ ID NO:1 (BHT-3021)
GCTGCTTCGCGATGTACGGGCCAGATATACGCGTTGACATTGATTATTGACTAGTTATTAATAGTAATCAATTAC
GGGGTCATTAGTTCATAGCCCATATATGGAGTTCCGCGTTACATAACTTACGGTAAATGGCCCGCCTGGCTGACC
GCCCAACGACCCCCGCCCATTGACGTCAATAATGACGTATGTTCCCATAGTAACGCCAATAGGGACTTTCCATTG
ACGTCAATGGGTGGAGTATTTACGGTAAACTGCCCACTTGGCAGTACATCAAGTGTATCATATGCCAAGTACGCC
CCCTATTGACGTCAATGACGGTAAATGGCCCGCCTGGCATTATGCCCAGTACATGACCTTATGGGACTTTCCTAC
TTGGCAGTACATCTACGTATTAGTCATCGCTATTACCATGGTGATGCGGTTTTGGCAGTACATCAATGGGCGTGG
ATAGCGGTTTGACTCACGGGGATTTCCAAGTCTCCACCCCATTGACGTCAATGGGAGTTTGTTTTGGCACCAAAA
TCAACGGGACTTTCCAAAATGTCGTAACAACTCCGCCCCATTGACGCAAATGGGCGGTAGGCGTGTACGGTGGGA
GGTCTATATAAGCAGAGCTCTCTGGCTAACTAGAGAACCCACTGCTTACTGGCTTATCGAAATTAATACGACTCA
CTATAGGGAGACCCAAGCTGGCTAGCGTAAGTATCAAGGTTACAAGACAGGTTTAAGGAGACCAATAGAAACTGG
GCTTGTCGAGACAGAGAAGACTCTTGCGTTTCTGATAGGCACCTATTGGTCTTACTGACATCCACTTTGCCTTTC
TCTCCACAGGCTTAAGCTTATGGCCTTTGTGAACCAACACCTGTGCGGCTCACACCTGGTGGAAGCTCTCTACCT
AGTGTGCGGGGAACGAGGCTTCTTCTACACACCCAAGACCCGCCGGGAGGCAGAGGACCTGCAGGTGGGGCAGGT
GGAGCTGGGCGGGGGCCCTGGTGCAGGCAGCCTGCAGCCCTTGGCCCTGGAGGGGTCCCTGCAGAAGCGTGGCAT
TGTGGAACAATGCTGTACCAGCATCTGCTCCCTCTACCAGCTGGAGAACTACTGCAACTAGCTCGAGTCTAGAGG
GCCCGTTTAAACCCGCTGATCAGCCTCGACTGTGCCTTCTAGTTGCCAGCCATCTGTTGTTTGCCCCTCCCCCGT
GCCTTCCTTGACCCTGGAAGGTGCCACTCCCACTGTCCTTTCCTAATAAAATGAGGAAATTGCATCGCATTGTCT
GAGTAGGTGTCATTCTATTCTGGGGGGTGGGGTGGGGCAGGACAGCAAGGGGGAGGATTGGGAAGACAATAGCAG
GCATGCTGGGGATGCGGTGGGCTCTATGGCTTCTACTGGGCGGTTTTATGGACAGCAAGCGAACCGGAATTGCCA
GCTGGGGCGCCCTCTGGTAAGGTTGGGAAGCCCTGCAAAGTAAACTGGATGGCTTTCTTGCGGCCAAGGATCTGA
TGGCGCAGGGGATCAAGCTCTGATCAAGAGACAGGATGAGGATGGTTTCGCATGATTGAACAAGATGGATTGCAC
GCAGGTTCTCCGGCAGCTTGGGTGGAGAGGCTATTCGGCTATGACTGGGCACAACAGACAATCGGCTGCTCTGAT
GCCGCCGTGTTCAGGCTGTCAGCGCAGGGGCGCCCGGTTCTTTTTGTCAAGACCGACCTGTCCGGTGCCCTGAAT
GAACTGCAAGACGAGGCAGCGCGGCTATCGTGGCTGGCCACGACGGGCGTTCCTTGCGCAGCTGTGCTCGACGTT
GTCACTGAAGCGGGAAGGGACTGGCTGCTATTGGGCGAAGTGCCGGGGCAGGATCTCCTGTCATCTCACCTTGCT
CCTGCCGAGAAAGTATCCATCATGGCTGATGCAATGCGGCGGCTGCATACGCTTGATCCGGCTACCTGCCCATTC
GACCACCAAGCGAAACATCGCATCGAGCGAGCACGTACTCGGATGGAAGCCGGTCTTGTCGATCAGGATGATCTG
GACGAAGAGCATCAGGGGCTCGCGCCAGCCGAACTGTTCGCCAGGCTCAAGGCGAGCATGCCCGACGGCGAGGAT
CTCGTCGTGACCCATGGCGATGCCTGCTTGCCGAATATCATGGTGGAAAATGGCAGGTTTTCTGGATTCATCGAC
TGTGGCCGGCTGGGTGTGGCGGACAGGTATCAGGACATAGCGTTGGCTACCCGTGATATTGCTGAAGAGCTTGGC
GGCGAATGGGCTGACAGGTTCCTCGTGCTTTACGGTATTGCGGCTCCCGATTCGCAGCGCATTGCCTTCTATAGG
CTTCTTGACGAGTTCTTCTGAATTATTAACGCTTACAATTTCCTGATGCGGTATTTTCTCCTTACGCATCTGTGC
GGTATTTCACACCGCATCAGGTGGCACTTTTCGGGGAAATGTGCGCGGAACCCCTATTTGTTTATTTTTCTAAAT
ACATTCAAATATGTATCCGCTCATGAGACAATAACCCTGATAAATGCTTCAATAATAGCACGTGCTAAAACTTCA
TTTTTAATTTAAAAGGATCTAGGTGAAGATCCTTTTTGATAATCTCATGACCAAAATCCCTTAACGTGAGTTTTC
GTTCCACTGAGCGTCAGACCCCGTAGAAAAGATCAAAGGATCTTCTTGAGATCCTTTTTTTCTGCGCGTAATCTG
CTGCTTGCAAACAAAAAAACCACCGCTACCAGCGGTGGTTTGTTTGCCGGATCAAGAGCTACCAACTCTTTTTCC
GAAGGTAACTGGCTTCAGCAGAGCGCAGATACCAAATACTGTTCTTCTAGTGTAGCCGTAGTTAGGCCACCACTT
CAAGAACTCTGTAGCACCGCCTACATACCTCGCTCTGCTAATCCTGTTACCAGTGGCTGCTGCCAGTGGCGATAA
GTCGTGTCTTACCGGGTTGGACTCAAGACGATAGTTACCGGATAAGGCGCAGCGGTCGGGCTGAACGGGGGGTTC
GTGCACACAGCCCAGCTTGGAGCGAACGACCTACACCGAACTGAGATACCTACAGCGTGAGCTATGAGAAAGCGC
CACGCTTCCCGAAGGGAGAAAGGCGGACAGGTATCCGGTAAGCGGCAGGGTCGGAACAGGAGAGCGCACGAGGGA
GCTTCCAGGGGGAAACGCCTGGTATCTTTATAGTCCTGTCGGGTTTCGCCACCTCTGACTTGAGCGTCGATTTTT
GTGATGCTCGTCAGGGGGGCGGAGCCTATGGAAAAACGCCAGCAACGCGGCCTTTTTACGGTTCCTGGCCTTTTG
CTGGCCTTTTGCTCACATGTTCTT

## Claims

1. A self-vector having a nucleic acid sequence at least 95% identical to SEQ ID NO:1 (BHT-3021) for use in reducing the frequency of CD8 T cells reactive to proinsulin in a method for the treatment or prevention of insulin-dependent diabetes mellitus (IDDM) in a subject.

2. The self-vector for the use of claim 1 which is SEQ ID NO:1 (BHT-3021).

3. The self-vector for the use of claim 1, wherein the self-vector forms a composition also comprising a pharmaceutically acceptable carrier.

4. The self-vector for the use of claim 3, wherein the composition is endotoxin-free.

5. The self-vector for the use of claim 3 or claim 4, wherein the pharmaceutically acceptable carrier comprises an adjuvant.

6. The self-vector for the use of claim 1 or claim 2, wherein the self-vector is administered intramuscularly.

7. The self-vector for the use of claim 1, wherein the subject has IDDM.

8. The self-vector for the use of claim 1 wherein the method comprises detecting a reduction in the frequency of CD8 T cells reactive to proinsulin.

## Patentansprüche

1. Eigenvektor, der eine Nucleinsäuresequenz aufweist, die zumindest zu 95 % mit Seq.-ID Nr. 1 (BHT-3021) identisch ist, zur Verwendung bei der Verringerung der Häufigkeit von gegenüber Proinsulin reaktiven CD8-T-Zellen in einem Verfahren zur Behandlung oder Prävention von insulinabhängigem Diabetes mellitus (IDDM) bei einem Individuum.

2. Eigenvektor zur Verwendung nach Anspruch 1, bei dem es sich um Seq.-ID Nr. 1 (BHT-3021) handelt.

3. Eigenvektor zur Verwendung nach Anspruch 1, wobei der Eigenvektor eine Zusammensetzung bildet, die auch einen pharmazeutisch annehmbaren Träger umfasst.

4. Eigenvektor zur Verwendung nach Anspruch 3, wobei die Zusammensetzung frei von Endotoxin ist.

5. Eigenvektor zur Verwendung nach Anspruch 3 oder Anspruch 4, wobei der pharmazeutisch annehmbare Träger ein Adjuvans umfasst.

6. Eigenvektor zur Verwendung nach Anspruch 1 oder Anspruch 2, wobei der Eigenvektor intramuskulär verabreicht wird.

7. Eigenvektor zur Verwendung nach Anspruch 1, wobei das Individuum IDDM hat.

8. Eigenvektor zur Verwendung nach Anspruch 1, wobei das Verfahren das Nachweisen einer Verringerung der Häufigkeit von gegenüber Proinsulin reaktiven CD8-T-Zellen umfasst.

## Revendications

1. Auto-vecteur possédant une séquence d'acide nucléique au moins 95 % identique à SEQ ID NO: 1 (BHT-3021) destiné à être utilisé pour réduire la fréquence des cellules T CD8 réactives pour la proinsuline dans un procédé de traitement ou de prévention du diabète insulinodépendant (DID) chez un sujet.

2. Auto-vecteur destiné à être utilisé selon la revendication 1 qui est SEQ ID NO: 1 (BHT-3021).

3. Auto-vecteur destiné à être utilisé selon la revendication 1, où l'auto-vecteur forme une composition comprenant également un véhicule pharmaceutiquement acceptable.

4. Auto-vecteur destiné à être utilisé selon la revendication 3, où la composition est exempte d'endotoxines.

5. Auto-vecteur destiné à être utilisé selon la revendication 3 ou la revendication 4, où le véhicule pharmaceutiquement acceptable comprend un adjuvant.

6. Auto-vecteur destiné à être utilisé selon la revendication 1 ou la revendication 2, où l'auto-vecteur est administré par voie intramusculaire.

7. Auto-vecteur destiné à être utilisé selon la revendication 1, où le sujet souffre de DID.

8. Auto-vecteur destiné à être utilisé selon la revendication 1, où le procédé comprend la détection d'une réduction de la fréquence des cellules T CD8 réactives pour la proinsuline.
